# EUROPÄISCHE PATENTSCHRIFT

(11) **EP 2 392 248 B1**
(45) Veröffentlichungstag und Bekanntmachung des Hinweises auf die Patenterteilung: **06.02.2019**
(21) Anmeldenummer: 11167891.8
(22) Anmeldetag: 27.05.2011
(51) Int. Cl.: A61B 1/018, A61B 1/06, G02B 23/24, A61B 1/07, A61B 1/00

(54) **Hülle für Bildübertragungsvorrichtung und Endoskop damit**
Sleeve for image transmission apparatus and endoscope with same
Enveloppe pour dispositif de transfert d'images et endoscope l'utilisant

(30) Priorität: 01.06.2010 DE 102010022430
(43) Veröffentlichungstag der Anmeldung: 07.12.2011
(73) Patentinhaber: Karl Storz SE & Co. KG, 78532 Tuttlingen (DE)
(72) Erfinder: Dahmen, Jan, 78532 Tuttlingen (DE)

(56) Entgegenhaltungen:
- WO-A1-90/08498
- DE-B3-102006 017 683
- US-A- 5 051 824
- US-A1- 2009 264 706
- US-B1- 6 478 730

## Beschreibung

Die vorliegende Erfindung bezieht sich auf eine Hülle für eine Bildübertragungsvorrichtung, insbesondere auf ein Endoskop mit besagter Hülle.

Das Sichtfeld eines Endoskops ist der vom unbewegten Endoskop erfasste bzw. mittels des unbewegten Endoskops erfassbare Raumbereich. Das Sichtfeld kann durch den Raumwinkel, den der erfasste Raumbereich bezogen auf das distale Ende des Endoskops einnimmt, durch die Blickrichtung und durch den Winkelabstand gegenüberliegender Grenzen des Sichtfelds charakterisiert werden. Die Blickrichtung ist die auf das distale Ende des Endoskops bezogene Richtung, in der Gegenstände liegen, die bei Betrachtung durch das Endoskop in der Mitte des erfassten Bilds liegen oder auf die Mitte eines lichtempfindlichen Sensors bzw. Bildsensors abgebildet werden.

Verschiedene Verwendungen bzw. Anwendungen eines Endoskops, insbesondere verschiedene medizinische Verwendungen, erfordern in der Regel verschiedene Sichtfelder, insbesondere verschiedene Blickrichtungen und verschiedene Größen der Sichtfelder. Es existiert deshalb eine große Zahl unterschiedlicher Endoskope mit unterschiedlichen Blickrichtungen und unterschiedlichen Größen der Sichtfelder. In Krankenhäusern, Arztpraxen und anderen medizinischen Einrichtungen werden entsprechend viele verschiedene Endoskope bereitgehalten. Die Beschaffung, Instandhaltung und Bereithaltung zahlreicher verschiedener Endoskope - in der Regel jeweils in mehreren Exemplaren - erzeugt entsprechende Kosten.

Hierzu schlagen die Druckschriften US 2009/0264706 A1, US 5,051,824 A, DE 10 2006 017 683 B3 und US 6,478,730 B1 jeweils eine Sichtfeldvorrichtung für eine Bildübertragungsvorrichtung vor, die einen Kanal aufweist, der das proximale Ende und das distale Ende der Sichtfeldvorrichtung verbindet und die Bildübertragungsvorrichtung aufnehmen kann, wobei eine Einrichtung am distalen Ende des Kanals ausgebildet ist, um das Sichtfeld der Bildübertragungsvorrichtung zu beeinflussen.

In WO 90/08498 A1 ist ein starres Videoendoskop mit einem inneren Körperbauteil B und einer hitzesterilisierbaren Hülle S beschrieben (Seite 1, Zeilen 3 bis 11; Seite 6, Zeilen 26 bis 30; Figuren 1, 2). Die hitzesterilisierbare Hülle S umfasst ein zylindrisches Gehäuse 30 mit einem zieharmonikaförmigen Schlauch 36, der nahe dem proximalen Ende des Gehäuses 30 angeordnet ist und über ein Anschlusskabel 22 des inneren Körperbauteils B gezogen werden kann (Seite 8, Zeilen 24 bis 33; Figur 7).

Eine Aufgabe der vorliegenden Erfindung besteht darin, den Aufwand für Beschaffung und Bereithaltung von Endoskopen mit zahlreichen verschiedenen Sichtfeldern zu verringern.

Diese Aufgabe wird durch die Gegenstände des unabhängigen Anspruchs gelöst.

Weiterbildungen sind in den abhängigen Ansprüchen angegeben.

Einige Ausführungsformen der vorliegenden Erfindung beruhen auf der Idee, eine Sichtfeldvorrichtung für eine Bildübertragungsvorrichtung, insbesondere für ein Endoskop, zu schaffen, die einen Kanal, der das proximale Ende und das distale Ende der Sichtfeldvorrichtung verbindet, zum Aufnehmen der Bildübertragungsvorrichtung und eine optische Einrichtung am distalen Ende des Kanals, die ausgebildet ist, um zumindest entweder das Sichtfeld oder die Fokussierung der Bildübertragungsvorrichtung zu beeinflussen, umfasst.

Mit einer Beeinflussung des Sichtfelds der Bildübertragungsvorrichtung ist keine lediglich beschneidende Einschränkung des Sichtfelds bei unveränderter Position und Größe des Bilds eines Gegenstands gemeint. Eine Beeinflussung des Sichtfelds bedeutet vielmehr gleichzeitig eine Veränderung der Größe und/oder eine Verschiebung der Position des Bilds eines bestimmten Gegenstands. Beispielsweise beeinflusst eine Glassscheibe oder ein Fenster oder ein Deckglas mit parallelen ebenen oder auch parallelen gekrümmten Grenzflächen das Sichtfeld nicht oder nur unwesentlich.

Mit ein und derselben Bildübertragungsvorrichtung können nun alternativ verschiedene Sichtfeldvorrichtungen kombiniert werden, um Endoskope mit verschiedenen Sichtfeldern zu bilden. Eine größere Anzahl verschiedener Sichtfeldvorrichtungen kann aufgrund ihres vergleichsweise einfachen Aufbaus kostengünstig beschafft und bereitgehalten werden. Jede Bildübertragungsvorrichtung kann mit mehreren verschiedenen Sichtfeldvorrichtungen kombiniert werden.

Bei einer Beschädigung oder Verschmutzung der Sichtfeldvorrichtung kann diese leicht ausgetauscht werden, die Bildübertragungsvorrichtung kann weiterverwendet werden. Insbesondere kann die Sichtfeldvorrichtung nach jedem medizinischen Einsatz ausgetauscht und beispielsweise autoklaviert werden. Auch die Bildübertragungsvorrichtung kann leicht austauschbar sein, beispielsweise um im Fall eines Defekts durch eine neue Bildübertragungsvorrichtung ersetzt zu werden, oder um durch eine Bildübertragungsvorrichtung mit anderen spektralen Eigenschaften ersetzt zu werden.

Die Anzahl der bereitzuhaltenden Bildübertragungsvorrichtungen wird dadurch drastisch reduziert. Selbst wenn die Kosten für die Herstellung bzw. Beschaffung der einzelnen Bildübertragungsvorrichtungen in der gleichen Größenordnung wie die eines herkömmlichen Endoskops liegen, können die Gesamtkosten drastisch reduziert werden, wenn die Herstellungskosten der Sichtfeldvorrichtungen deutlich niedriger sind als die eines vollständigen Endoskops, da die Anzahl der erforderlichen Bildübertragungsvorrichtungen deutlich geringer ist als die Anzahl der herkömmlich erforderlichen Endoskope.

Die optische Einrichtung am distalen Ende des Kanals kann alternativ oder zusätzlich zum Sichtfeld die Fokussierung der Bildübertragungseinrichtung beeinflussen. Gegenstände, die von der Bildübertragungseinrichtung auf eine gegebene Bildfläche scharf abgebildet werden, liegen in einer Fläche. Diese Fläche kann, abhängig von der optischen Einrichtung, eben oder gewölbt, symmetrisch oder asymmetrisch zur optischen Achse der optischen Einrichtung sein. Mit einer Beeinflussung der Fokussierung ist insbesondere eine Beeinflussung oder Veränderung des Ort und/oder der Form dieser Fläche gemeint.

Die optische Einrichtung der Sichtfeldvorrichtung ist insbesondere ausgebildet, um Licht zumindest entweder zu brechen oder zu reflektieren oder zu beugen. Insbesondere umfasst die optische Einrichtung zumindest entweder ein Prisma oder einen Spiegel oder eine Linse. Mit diesen verhältnismäßig einfachen Bauteilen kann die Sichtfeldvorrichtung kostengünstig herstellbar sein.

Die optische Einrichtung der Sichtfeldvorrichtung ist insbesondere ausgebildet, um die Richtung des Beleuchtungs- und/oder des Abbildungsstrahlengangs zu verändern oder die Divergenz von Licht im Beleuchtungs- und/oder Abbildungsstrahlengang zu verändern.

Der Kanal der Sichtfeldvorrichtung ist ausgebildet, um eine Rotation einer Bildübertragungsvorrichtung um ihre Längsachse in dem Kanal zu ermöglichen. Dazu sind die Querschnitte der Bildübertragungsvorrichtung und des Kanals aufeinander abgestimmt. Beispielsweise durch kreisförmige Querschnitte mit entsprechenden Radien können eine spielarme und gleichzeitig reibungsarme Führung der Bildübertragungsvorrichtung im Kanal, eine Rotierbarkeit und eine axiale Verschiebbarkeit der Bildübertragungsvorrichtung im Kanal geschaffen werden. Dadurch kann bei einer Rotation der Sichtfeldvorrichtung um die Längsachse und einer entsprechenden Variation der Blickrichtung auf einem Kegelmantel durch gleichzeitiges Festhalten der Bildübertragungsvorrichtung ein Kippen des Bilds verhindert werden. Das aus der Sichtfeldvorrichtung und der Bildübertragungsvorrichtung gebildete Endoskop kann somit unabhängig von der Blickrichtung immer ein aufrechtes Bild liefern.

Die Rotierbarkeit der Bildübertragungsvorrichtung im Kanal der Sichtfeldvorrichtung bedeutet insbesondere eine Rotierbarkeit um mehr als nur einen kleinen Winkel, beispielsweise um mindestens 90°, mindestens 180°, mindestens 270°, mindestens 360° oder um einen beliebigen Winkel.

Wenn die Sichtfeldvorrichtung eine Linse oder ein anderes optisch wirksames Bauelement umfasst, kann durch eine axiale Verschiebung der Bildübertragungsvorrichtung im Kanal der Sichtfeldvorrichtung die Fokussierung verändert bzw. die scharf abgebildete Ebene verschoben werden. Diese einfache Art der Fokussierung ermöglicht die Verwendung einer Optik mit einer großen Apertur, die ein entsprechend helles und rauscharmes Bild erzeugt. Im Vergleich zu einer herkömmlich oft verwendeten kleinen Apertur mit entsprechend hoher Schärfentiefe und ohne Fokussierung ermöglicht die beschriebene Fokussierung hellere und rauschärmere Bilder und/oder die Verwendung geringerer Beleuchtungsintensitäten.

Wenn ferner ein lichtempfindlicher Sensor der Bildübertragungseinrichtung - gegebenenfalls zusammen mit einem Stablinsensystem - gegenüber einer Linse, einem Objektiv bzw. einem optisch wirksamen Bauelement am distalen Ende der Bildübertragungsvorrichtung verschiebbar ist, kann zumindest entweder der Fokus oder die Brennweite bzw. die Größe des Sichtfelds verändert werden. Für eine Veränderung der Brennweite sind beispielsweise zumindest eine erste Linse bzw. ein erster Teil eines Objektivs mit der Sichtfeldvorrichtung gekoppelt und zumindest eine zweite Linse bzw. ein zweiter Teil eines Objektivs mit der Bildübertragungseinrichtung gekoppelt.

Alternativ kann die Sichtfeldvorrichtung mit einer Bildübertragungseinrichtung eingesetzt werden, die anstelle einer Bildübertragungseinrichtung ein Okular oder eine Kupplung zum Anschluss einer Kamera am proximalen Ende aufweist. Zur Übertragung eines Bilds vom distalen Ende zum proximalen Ende der Bildübertragungseinrichtung kann wie beim oben beschriebenen Beispiel ein Stablinsensystem vorgesehen sein. Alternativ ist beispielsweise ein geordnetes Bündel von Lichtwellenleitern zur Übertragung des Bilds zum Okular bzw. zur Kupplung vorgesehen. Das oben zur Veränderbarkeit von Fokus und/oder Brennweite durch Verschiebung von Sichtfeldvorrichtung und Bildübertragungseinrichtung relativ zu einander Ausgeführte gilt entsprechend für eine Bildübertragungseinrichtung mit einem Okular oder einer Kupplung.

Die Sichtfeldvorrichtung kann eine Justageeinrichtung zur justierbaren Festlegung der Position einer Bildübertragungsvorrichtung in Längsrichtung in dem Kanal aufweisen. Die Justageeinrichtung umfasst beispielsweise eine Stellschraube, die in Form einer Hülse oder Einstellmutter koaxial im Bereich des Schafts oder im Bereich einer Handhabungseinrichtung der Bildübertragungsvorrichtung angeordnet sein kann. Eine solche Justageeinrichtung kann eine gleichzeitig einfach vornehmbare und exakte Feinjustierung des Fokus bzw. der Lage der scharf abgebildeten Ebene ermöglichen.

Die Sichtfeldvorrichtung kann einen oder mehrere Lichtwellenleiter zur Übertragung von Beleuchtungslicht vom proximalen Ende zum distalen Ende der Sichtfeldvorrichtung zur Beleuchtung eines zu betrachtenden Objekts umfassen. Wenn die Sichtfeldvorrichtung mehrere Lichtwellenleiter zur Übertragung von Beleuchtungslicht umfasst, bilden diese insbesondere ein ungeordnetes Bündel, im Gegensatz zu einem zur Übertragung eines Bilds geeigneten und in vielen Fällen verwendeten geordneten Bündels von Lichtwellenleitern.

Ferner kann die Sichtfeldvorrichtung am distalen Ende eine Lichtaustrittseinrichtung zum Leiten von Beleuchtungslicht in einen Raumbereich außerhalb der Sichtfeldvorrichtung umfassen. Die Lichtaustrittseinrichtung kann ein Beleuchtungsfenster, und/oder ein Prisma und/oder eine Linse und/oder einen Spiegel umfassen. Die Lichtaustrittseinrichtung kann mit dem distalen Ende von einem oder mehreren in die Sichtfeldvorrichtung integrierten Lichtwellenleitern zur Übertragung von Beleuchtungslicht gekoppelt sein. Alternativ kann die Lichtaustritteinrichtung zur Kopplung mit einem oder mehreren in eine Bildübertragungsvorrichtung integrierten Lichtwellenleitern zur Übertragung von Beleuchtungslicht ausgebildet sein.

Die in die Sichtfeldvorrichtung integrierte Lichtaustrittseinrichtung, ggf. kombiniert mit einem oder mehreren in die Sichtfeldvorrichtung integrierten Lichtwellenleitern zum Übertragen von Beleuchtungslicht, ermöglicht eine weitgehende oder vollständige Anpassung des beleuchteten Raumbereichs an das Sichtfeld.

Die optische Einrichtung am distalen Ende des Kanals der Sichtfeldvorrichtung kann nach außen bzw. nach distal fluiddicht abgedichtet sein. Dazu kann ein fluiddicht eingesetztes Deckglas oder eine fluiddichte Verbindung zwischen der optischen Einrichtung selbst oder dem äußerst distalen Element der optischen Einrichtung einerseits und beispielsweise rohrartigen Strukturen der Sichtfeldvorrichtung andererseits vorgesehen sein. Zusätzlich kann eine fluiddichte Abdichtung der optischen Einrichtung nach proximal bzw. zum Kanal hin vorgesehen sein. Dazu ist beispielsweise ein fluiddicht eingesetztes weiteres Deckglas oder eine fluiddichte Verbindung zwischen der optischen Einrichtung selbst oder einem äußerst proximalen Element der optischen Einrichtung einerseits und der Innenwand des Kanals andererseits vorgesehen.

Die fluiddichte Abdichtung der optischen Einrichtung ermöglicht ein Autoklavieren der Sichtfeldvorrichtung ohne ein Eindringen von Feuchtigkeit in die optische Einrichtung. Die optischen Eigenschaften der optischen Einrichtung können somit auch nach zahlreichen Autoklavierungszyklen uneingeschränkt erhalten bleiben. Wenn die optische Einrichtung nach proximal bzw. zum Kanal hin nicht fluiddicht abgedichtet ist, kann während des Autoklavierens ein Dichtstopfen am proximalen Ende des Kanals eingesetzt werden, um ein Eindringen von Feuchtigkeit in den Kanal und in die optische Einrichtung zu vermeiden. Ein solcher Dichtstopfen kann einen oder mehrere O-Ringe oder andere Dichteinrichtungen umfassen.

Die Sichtfeldvorrichtung kann einen Schaft aufweisen, der so lang wie ein Schaft der Bildübertragungsvorrichtung oder auch kürzer als dieser sein kann. Die Sichtfeldvorrichtung kann als geschlossene, insbesondere fluiddichte Hülle für die Bildübertragungsvorrichtung ausgebildet sein. In diesem Fall kann die Sichtfeldvorrichtung ein Autoklavieren der Bildübertragungsvorrichtung zwischen zwei Verwendungen überflüssig machen. Dies ist auch erzielbar, wenn die Sichtfeldvorrichtung die Bildübertragungsvorrichtung zwar nicht in Form einer Hülle vollständig und fluiddicht umschließt aber stattdessen durch ein Steriltuch zum Abdecken von sonst nicht durch die Sichtfeldvorrichtung bedeckten bzw. umhüllten Teilen der Bildübertragungsvorrichtung ergänzt wird.

Die beschriebenen Sichtfeldvorrichtungen können für starre oder flexible Bildübertragungsvorrichtungen ausgebildet sein, insbesondere für Videoendoskope, Endoskope mit Stablinsenoptik oder mit einem geordneten Bündel von Lichtwellenleitern zur Übertragung eines Bilds von distal nach proximal. Dazu kann die Sichtfeldvorrichtung entsprechend starr oder flexibel ausgebildet sein. Die beschriebenen Sichtfeldvorrichtungen sind insbesondere zur Verwendung mit medizinischen Endoskopen ausgebildet oder ausgebildet, um mit einer Bildübertragungsvorrichtung zu einem medizinischen Endoskop kombiniert zu werden. Dazu sind die beschriebenen Sichtfeldvorrichtungen insbesondere autoklavierbar oder auf andere Weise sterilisierbar oder aber als Wegwerfartikel bzw. Einmalartikel mit entsprechend geringen Herstellungskosten ausgebildet.

Die beschriebenen Sichtfeldvorrichtungen können jedoch auch für nicht-medizinische technische Verwendungen bzw. zur Verwendung mit nicht-medizinischen technischen Endoskopen bzw. Boroskopen ausgebildet sein. Durch die beschriebenen Sichtfeldvorrichtungen kann gleichzeitig eine Verschmutzung oder auch eine Beschädigung einer Bildübertragungsvorrichtung, insbesondere eines Endoskops, vermieden werden, so dass dieses nach Austausch der Sichtfeldvorrichtung sofort weiterverwendet werden kann, ohne vorher aufwendig gereinigt werden zu müssen.

Jede der beschriebenen Sichtfeldvorrichtungen kann für eine Bildübertragungsvorrichtung ausgebildet sein, die ein Endoskop mit allen Merkmalen, Eigenschaften und Funktionalitäten eines Endoskops im herkömmlichen Sinn des Begriffs ist. Eine solche Bildübertragungsvorrichtung kann auch ohne eine der beschriebenen Sichtfeldvorrichtungen als Endoskop bei medizinischen oder nicht-medizinischen, technischen endoskopischen Untersuchungen oder Eingriffen eingesetzt werden. Zu diesem Zweck ist die Bildübertragungsvorrichtung beispielsweise autoklavierbar, weist Tasten oder andere Elemente zur Bedienung durch einen Benutzer auf und liefert ein scharfes Bild von Gegenständen.

Alternativ kann jede der beschriebenen Sichtfeldvorrichtungen für eine Bildübertragungsvorrichtung ausgebildet sein, die erst in Kombination mit der Sichtfeldvorrichtung alle für eine Verwendung bei medizinischen oder nicht-medizinischen technischen endoskopischen Untersuchungen oder Eingriffen erforderlichen Eigenschaften und Funktionen aufweist. Beispielsweise ist die Bildübertragungsvorrichtung nicht autoklavierbar oder liefert kein scharfes Bild, wenn sie nicht mit einer Sichtfeldvorrichtung kombiniert ist.

Weitere Ausführungsformen der vorliegenden Erfindung beruhen auf der Idee, eine Bildübertragungsvorrichtung mit einem Schaft und zumindest entweder einer Einrichtung zum optischen Übertragen eines Bilds von einem distalen Ende zu einem proximalen Ende des Schafts oder einem Bildsensor zum Wandeln eines optischen Bilds in ein Bildsignal zu schaffen, wobei der Schaft ausgebildet ist, um in einen Kanal in einer Sichtfeldvorrichtung eingesetzt zu werden, die am distalen Ende des Kanals eine optische Einrichtung aufweist, welche das Sichtfeld des Endoskops beeinflusst. Die Bildübertragungsvorrichtung ist insbesondere ausgebildet, um ein scharfes Bild eines Gegenstands zu erzeugen oder nur dann zu erzeugen, wenn die Bildübertragungsvorrichtung in einen Kanal von einer der oben beschriebenen Sichtfeldvorrichtungen eingesetzt ist. Die Bildübertragungsvorrichtung kann am distalen Ende des Schafts einen fluiddicht eingesetzten transparenten Verschluss aufweisen, insbesondere ein Deckglas oder ein Beobachtungsfenster aus einem transparenten Material.

Weitere Ausführungsformen der vorliegenden Erfindung beruhen auf der Idee, ein Endoskop mit einer der oben beschriebenen Sichtfeldvorrichtungen und einer in den Kanal der Sichtfeldvorrichtung einsetzbaren Bildübertragungsvorrichtung, insbesondere der oben beschriebenen Bildübertragungsvorrichtung, zu schaffen, wobei die Bildübertragungsvorrichtung zumindest entweder eine Einrichtung zum optischen Übertragen eines Bilds von einem distalen Ende des Endoskops zu einem proximalen Ende des Endoskops oder einen Bildsensor zum Wandeln eines optischen Bilds in ein Bildsignal aufweist.

Weitere Ausführungsformen beruhen auf der Idee, bei einem Verfahren zum Bereitstellen eines Endoskops für eine nachfolgende Verwendung des Endoskops ein bei der nachfolgenden Verwendung erforderliches Sichtfeld zu bestimmen, eine Sichtfeldvorrichtung mit dem bestimmten Sichtfeld auszuwählen und eine Bildübertragungsvorrichtung mit der ausgewählten Sichtfeldvorrichtung zu kombinieren, insbesondere in einen Kanal der ausgewählten Sichtfeldvorrichtung einzuführen, um ein Endoskop zu bilden.

Weitere Ausführungsformen beruhen auf der Idee, bei einem Verfahren zum Autoklavieren einer Sichtfeldvorrichtung für eine Bildübertragungsvorrichtung, mit einem Kanal, der das proximale Ende und das distale Ende der Sichtfeldvorrichtung verbindet, zum Aufnehmen einer Bildübertragungsvorrichtung, und einer optischen Einrichtung am distalen Ende des Kanals, die ausgebildet ist, um das Sichtfeld des Endoskops zu beeinflussen, den Kanal am proximalen Ende fluiddicht zu verschließen, nach dem fluiddichten Verschließen die Sichtfeldvorrichtung zu autoklavieren und nach dem Autoklavieren den Kanal am proximalen Ende zu öffnen.

Die vorstehend beschriebenen Verfahren werden insbesondere mit einer der oben beschriebenen Sichtfeldvorrichtungen und/oder mit der oben beschriebenen Bildübertragungsvorrichtung ausgeführt. Die oben beschriebenen Sichtfeldvorrichtungen und die oben beschriebene Bildübertragungsvorrichtung sind insbesondere zur Durchführung von einem der oben genannten Verfahren ausgebildet und geeignet.

### Kurzbeschreibung der Figuren

Nachfolgend werden Ausführungsformen anhand der beigefügten Figuren näher erläutert. Es zeigen:

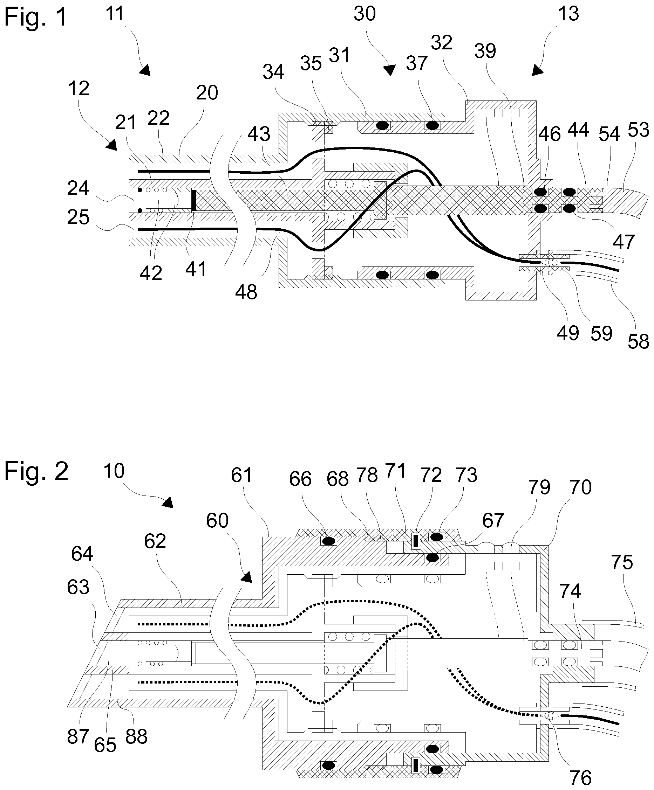

- Figur 1: eine schematische Darstellung einer Bildübertragungsvorrichtung;
- Figur 2: eine schematische Darstellung einer Sichtfeldvorrichtung für eine Bildübertragungsvorrichtung;

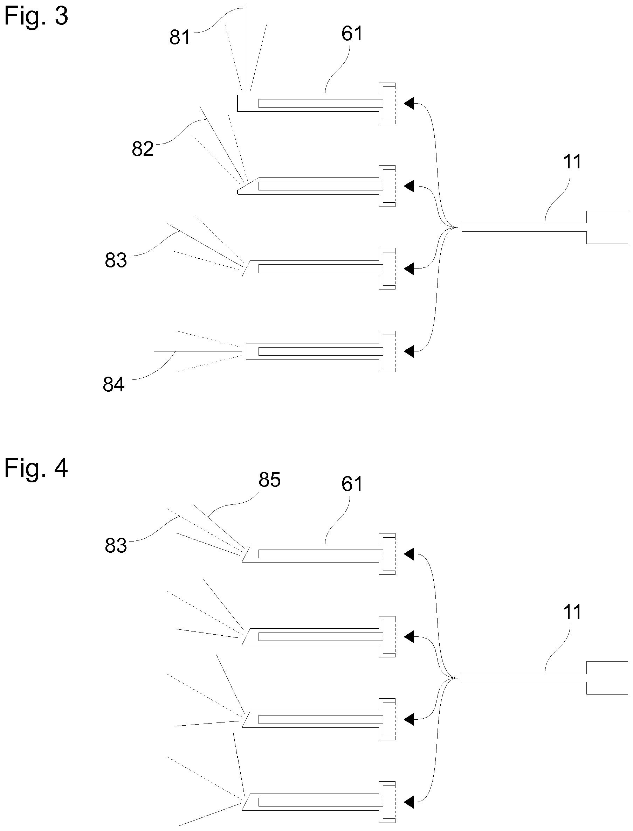

- Figur 3: eine schematische Darstellung einer Kombination einer Bildübertragungsvorrichtung mit einer von mehreren Sichtfeldvorrichtungen;
- Figur 4: eine schematische Darstellung einer Kombination einer Bildübertragungsvorrichtung mit einer von mehreren Sichtfeldvorrichtungen;

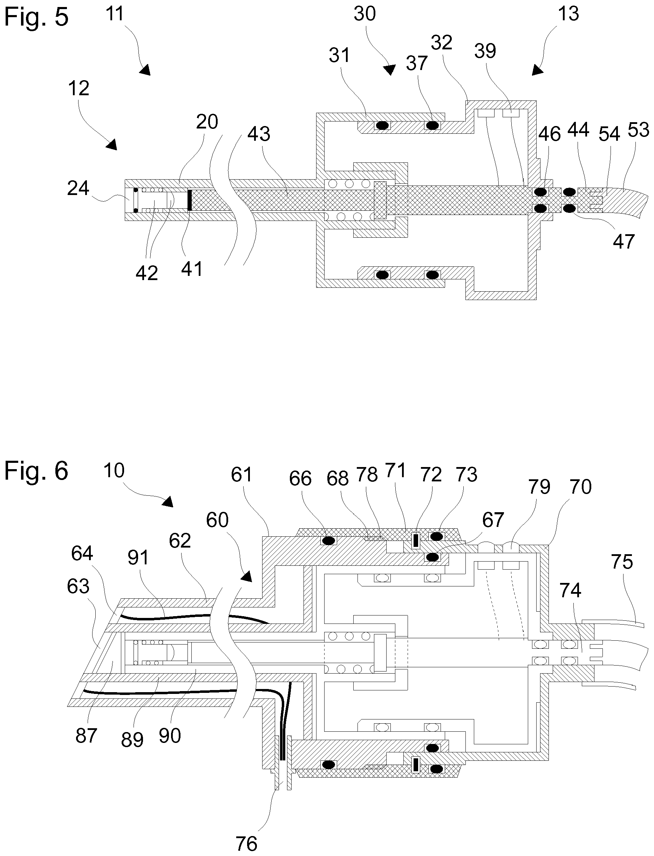

- Figur 5: eine schematische Darstellung einer Bildübertragungsvorrichtung;
- Figur 6: eine schematische Darstellung einer Sichtfeldvorrichtung für eine Bildübertragungsvorrichtung;

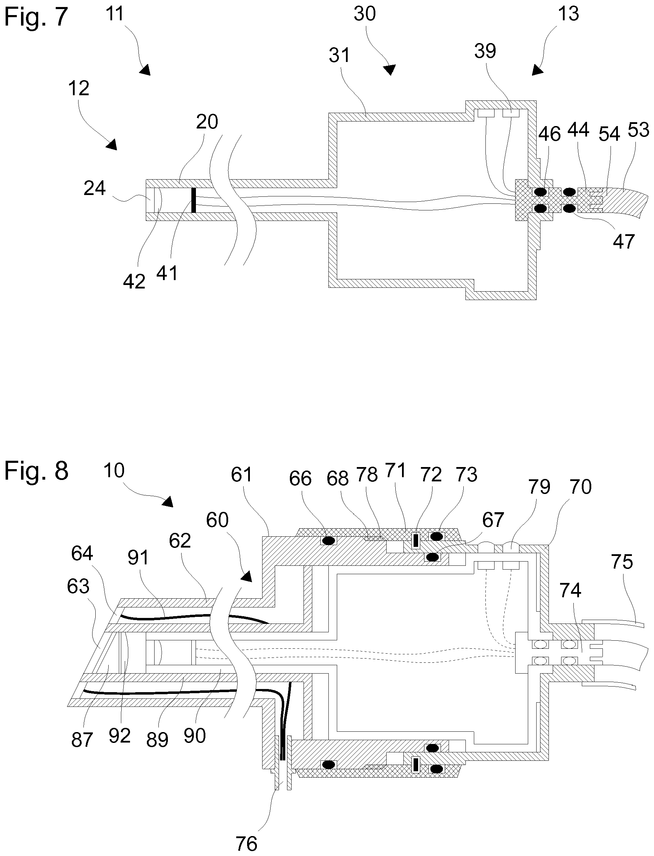

- Figur 7: eine schematische Darstellung einer Bildübertragungsvorrichtung;
- Figur 8: eine schematische Darstellung einer Sichtfeldvorrichtung für eine Bildübertragungsvorrichtung;

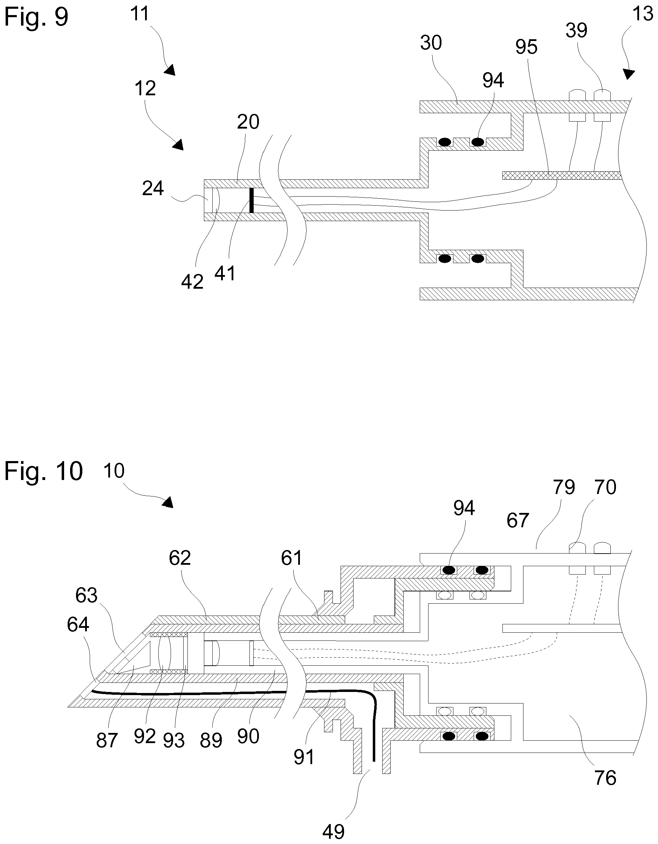

- Figur 9: eine schematische Darstellung einer Bildübertragungsvorrichtung;
- Figur 10: eine schematische Darstellung einer Sichtfeldvorrichtung für eine Bildübertragungsvorrichtung;

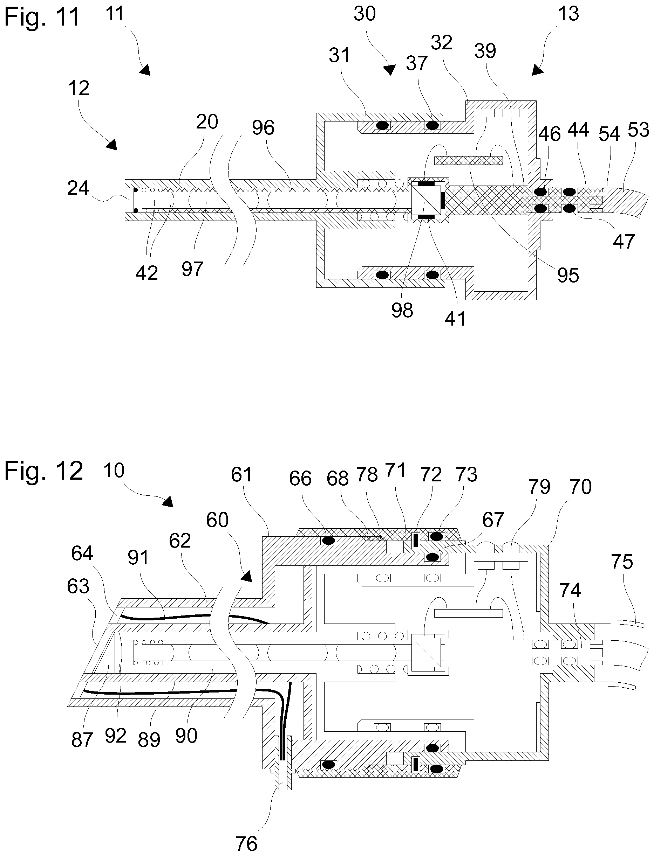

- Figur 11: eine schematische Darstellung einer Bildübertragungsvorrichtung;
- Figur 12: eine schematische Darstellung einer Sichtfeldvorrichtung für eine Bildübertragungsvorrichtung;

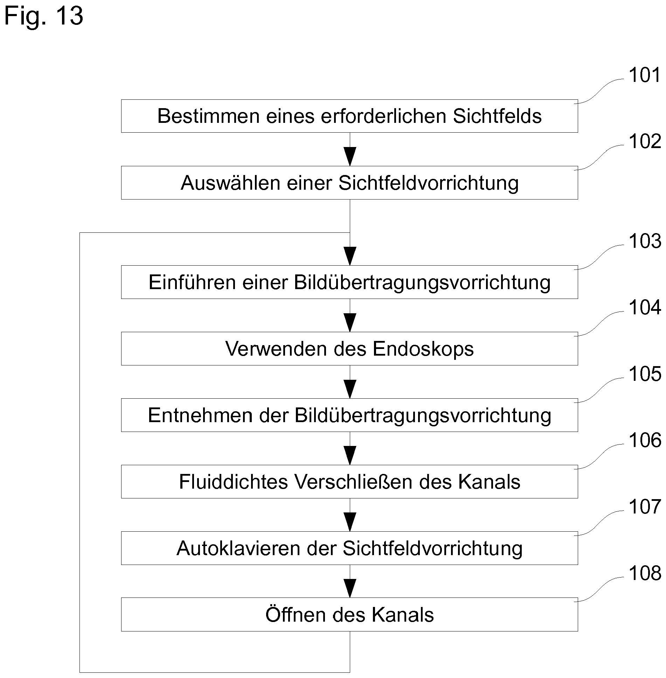

- Figur 13: ein schematisches Flussdiagramm eines Verfahrens zum Bereitstellen und zum Autoklavieren eines Endoskops.

### Beschreibung der Ausführungsformen

Jede der Figuren 1 bis 10 zeigt eine schematische Darstellung eines Endoskops oder von Bestandteilen eines Endoskops oder des Zusammenführens von Bestandteilen eines Endoskops. Dabei zeigt jede der Figuren 1 bis 10 im Wesentlichen eine Darstellung eines Schnitts entlang einer Ebene, die eine Längsachse des Endoskops enthält. Die Darstellung von Lichtwellenleitern oder elektrischen Leitungen kann von einer reinen Schnittdarstellung abweichen, um die räumliche Anordnung derselben zu verdeutlichen. Das Endoskop oder Bestandteile des Endoskops können teilweise oder vollständig rotationssymmetrisch zur Längsachse des Endoskops sein.

In jeder der Figuren 1 bis 10 sind eine Bildübertragungsvorrichtung oder zumindest Teile einer Bildübertragungsvorrichtung dargestellt. Im Sinne einer übersichtlichen Darstellung sind jedoch Bestandteile und Merkmale der Bildübertragungsvorrichtungen teilweise nur in den Figuren 1, 5, 7 und 9 mit Bezugszeichen versehen. In den Figuren 2, 6, 8 und 10 sind viele Bestandteile und Merkmale der Bildübertragungsvorrichtungen nicht mit Bezugszeichen versehen, um die Darstellungen nicht zu überfrachten.

Figur 1 zeigt eine Bildübertragungsvorrichtung 11. Die Bildübertragungsvorrichtung 11 kann ein Endoskop mit allen Merkmale und Eigenschaften - insbesondere Gebrauchseigenschaften und Funktionalitäten - eines Endoskops im herkömmlichen Sinn des Begriffs sein. Insbesondere kann die Bildübertragungsvorrichtung ausgebildet und geeignet sein, um ohne eine Sichtfeldvorrichtung, wie sie nachfolgend anhand der weiteren Figuren beschrieben werden, bei medizinischen oder nicht-medizinischen, technischen endoskopischen Untersuchungen oder Eingriffen eingesetzt zu werden. Zu diesem Zweck ist die Bildübertragungsvorrichtung beispielsweise autoklavierbar, weist Tasten oder andere Elemente zur Bedienung durch einen Benutzer auf und liefert ein scharfes Bild von Gegenständen.

Alternativ kann die Bildübertragungsvorrichtung 11 ausgebildet sein, um erst in Kombination mit einer Sichtfeldvorrichtung, wie sie nachfolgend anhand der weiteren Figuren beschrieben werden, alle für eine Verwendung bei medizinischen oder nicht-medizinischen technischen endoskopischen Untersuchungen oder Eingriffen erforderlichen Eigenschaften und Funktionen aufzuweisen. Beispielsweise ist die Bildübertragungsvorrichtung 11 nicht autoklavierbar oder liefert kein scharfes Bild, wenn sie nicht mit einer Sichtfeldvorrichtung kombiniert ist.

Die Bildübertragungsvorrichtung 11 weist ein distales Ende 12 und ein proximales Ende 13 auf. Ein Schaft 20 der Bildübertragungsvorrichtung 11 weist ein Innenrohr 21 und ein Außenrohr 22 auf. Am distalen Ende 12 weist der Schaft 20 ein Beobachtungsfenster 24 und ein oder mehrere Beleuchtungsfenster 25 auf, die jeweils Glas oder ein anderes transparentes Material aufweisen. Das Beobachtungsfenster 24 und das Beleuchtungsfenster 25 sind insbesondere voneinander optisch getrennt, beispielsweise durch den distalen Rand des Innenrohrs 21. Alternativ ist ein einziges Fenster vorgesehen, das die Funktionen des Beobachtungsfensters 24 und des oder der Beleuchtungsfenster 25 übernimmt.

Am proximalen Ende 13 weist die Bildübertragungsvorrichtung 11 eine Handhabungseinrichtung 30 mit einem distalen Gehäuseteil 31 und einem proximalen Gehäuseteil 32 auf. Das distale Gehäuseteil 31 der Handhabungseinrichtung 30 ist mit dem proximalen Ende des Außenrohrs 22 des Schafts 20 mechanisch verbunden, insbesondere gefügt. Auch das proximale Ende des Innenrohrs 21 ist mit dem distalen Gehäuseteil 31 der Handhabungseinrichtung 30 mechanisch verbunden, in diesem Beispiel durch eine Verschraubung 34 mit einer Kontermutter 35.

Das distale Gehäuseteil 31 und das proximale Gehäuseteil 32 der Handhabungseinrichtung 30 sind relativ zueinander bezogen auf die Längsachse der Bildübertragungsvorrichtung 11 und insbesondere des Schafts 20 rotierbar und/oder axial verschiebbar. Zwischen dem distalen Gehäuseteil 31 und dem proximalen Gehäuseteil 32 der Handhabungseinrichtung 30 sind ein oder mehrere O-Ringe 37 zur fluiddichten Abdichtung des Innenraums der Handhabungseinrichtung 30 und des Schafts 20 einerseits gegenüber der Umgebung andererseits vorgesehen.

An der Handhabungseinrichtung 30, insbesondere am distalen Gehäuseteil 32 der Handhabungseinrichtung 30, können Schalter, Taster oder andere Bedienelemente vorgesehen sein, mit denen beispielsweise die Beleuchtung, die Erfassung von Bildern oder deren Speicherung steuerbar sind. Beispielhaft sind in Figur 1 Magnetsensoren 39 dargestellt, die über elektrische Leitungen mit dem Sensorträger 43 und über diesen mit dem Steckverbinder 44 verbunden sind.

Am distalen Ende des Schafts 20 sind im Innenrohr 21 ein lichtempfindlicher Sensor 41 und ein Objektiv 42 angeordnet. Das Objektiv 42 bildet Gegenstände nahe dem distalen Ende 12 der Bildübertragungsvorrichtung 11 auf den lichtempfindlichen Sensor 41 ab. Der lichtempfindliche Sensor 41 ist am distalen Ende eines Sensorträgers 43 angeordnet, der sich stabförmig im Innenrohr 21 des Schafts 20 und weiter bis zum proximalen Ende des proximalen Gehäuseteils 32 der Handhabungseinrichtung 30 erstreckt. Der Sensorträger 43 wird, wie in Figur 1 angedeutet, von einer Feder gegen das proximale Ende des proximalen Gehäuseteils 32 der Handhabungseinrichtung 30 gedrückt, wo er sich abstützt. Alternativ ist der Sensorträger 43 am proximalen Ende des proximalen Gehäuseteils 32 der Handhabungseinrichtung 30 befestigt.

Eine oder mehrere elektrische oder optische Signalleitungen im oder am Sensorträger 43 koppeln den lichtempfindlichen Sensor 41 mit einem Steckverbinder 44 am proximalen Ende der Handhabungseinrichtung 30. Ein O-Ring 46 ist zur fluiddichten Abdichtung zwischen dem Sensorträger 43 und dem proximalen Gehäuseteil 32 der Handhabungseinrichtung 30 vorgesehen. Ein weiterer O-Ring 47 ist zur fluiddichten Abdichtung zwischen dem Sensorträger 43 und einer nachfolgend anhand der Figur 2 beschriebenen Sichtfeldvorrichtung vorgesehen.

Lichtwellenleiter 48 sind zwischen dem Innenrohr 21 und dem Außenrohr 22 des Schafts 20 der Bildübertragungsvorrichtung 11 angeordnet. Die Lichtwellenleiter 48 erstrecken sich von dem oder den Beleuchtungsfenstern 25 bis zu einer Durchführung oder einem Steckverbinder 49 am proximalen Ende des proximalen Gehäuseteils 32 der Handhabungseinrichtung 30. Anstelle des als transparentes Bauteil eingesetzten Beleuchtungsfensters 25 können die distalen Enden der Lichtwellenleiter 48 am distalen Ende des Schafts 20 der Bildübertragungsvorrichtung 11 lediglich mittels eines (optional transparenten) Kitts eingekittet sein. Durch Überschleifen und Polieren der distalen Stirnfläche des Schafts 20 mit den Enden der Lichtwellenleiter 48 und dem Kitt ist eine optisch hochwertige Lichtaustrittsfläche erzielbar.

Ein Signalkabel 53 mit einem Steckverbinder 54 ist mit dem Steckverbinder 44 am proximalen Ende des Sensorträgers 43 verbindbar. Über das Signalkabel 53 können beispielsweise elektrische Signale und elektrische Leistung zwischen dem lichtempfindlichen Sensor 41 und einer in Figur 1 nicht dargestellten Einrichtung zur Aufbereitung und Darstellung von Bildern ausgetauscht werden.

Ein Lichtwellenleiterkabel 58 mit einem Steckverbinder 59 kann mit der Durchführung oder dem Steckverbinder 49 gekoppelt werden. Über das Lichtwellenleiterkabel 58, die Steckverbinder 59, 49 und die Lichtwellenleiter 48 kann Licht von einer externen und in Figur 1 nicht dargestellten Lichtquelle zum distalen Ende 12 der Bildübertragungsvorrichtung 11 geleitet werden, um Gegenstände nahe dem distalen Ende 12 der Bildübertragungsvorrichtung 11 zu beleuchten.

Die anhand der Figur 1 dargestellte Bildübertragungsvorrichtung 11 weist wie erwähnt einen lichtempfindlichen Sensor 41 auf, der an seiner lichtempfindlichen Oberfläche vorliegende Lichtintensitäten in analoge oder digitale elektrische oder auch optische Signale wandelt. Diese Signale stellen das durch das Objektiv 42 auf der lichtempfindlichen Oberfläche des lichtempfindlichen Sensors 41 erzeugte Bild in analoger oder digitaler Form dar. Die Signale werden über die erwähnten elektrischen Leitungen bzw. über optische Lichtwellenleiter im Sensorträger zum proximalen Ende 13 der Bildübertragungsvorrichtung und weiter über das Signalkabel 53 zu in Figur 1 nicht dargestellten Vorrichtungen zur Auswertung der Signale und Darstellung des Bilds (beispielsweise auf einem Bildschirm) übertragen.

Alternativ weist die Bildübertragungsvorrichtung 11 eine Stablinsenoptik, ein geordnetes Bündel von Lichtwellenleitern oder eine andere optische Einrichtung zur Übertragung des von dem Objektiv erzeugten Bilds zum proximalen Ende 13 der Bildübertragungsvorrichtung 11 auf. Das Bild kann dann am proximalen Ende 13 der Bildübertragungsvorrichtung 11 beispielsweise durch ein Okular betrachtet, von einem lichtempfindlichen Sensor in analoge oder digitale elektrische oder auch optische Signale gewandelt oder über ein geordnetes Bündel von Lichtwellenleitern weiter übertragen werden.

Wie erwähnt kann die Bildübertragungsvorrichtung 11 ausgebildet und geeignet sein, um unabhängig von der nachfolgend anhand der Figur 2 dargestellten Sichtfeldvorrichtung als Endoskop eingesetzt zu werden. Zu diesem Zweck sind insbesondere der lichtempfindliche Sensor 41 und das Objektiv 42 ausgebildet und geeignet, um ohne weitere Licht brechende oder reflektierende Einrichtungen ein scharfes Bild eines Gegenstands nahe dem distalen Ende 12 der Bildübertragungsvorrichtung 11 zu erfassen.

Alternativ ist die Bildübertragungsvorrichtung 11 ausgebildet, um nur in Kombination mit der nachfolgend anhand der Figur 2 dargestellten Sichtfeldvorrichtung als Endoskop verwendet zu werden. Zu diesem Zweck sind insbesondere der lichtempfindliche Sensor 41 und das Objektiv 42 ausgebildet und geeignet, um erst in Kombination mit einer weiteren Licht brechenden oder reflektierenden Einrichtung an der unten beschriebenen Sichtfeldvorrichtung ein scharfes Bild eines Gegenstands nahe dem distalen Ende 12 der Bildübertragungsvorrichtung 11 zu erfassen. Ferner kann in diesem Fall das Objektiv 42 entfallen.

Figur 2 zeigt eine schematische Darstellung einer Hülle 60 aus einer Sichtfeldvorrichtung 61 und einem proximalen Hüllenteil 70 für die oben anhand der Figur 1 dargestellte Bildübertragungsvorrichtung 11. Die Sichtfeldvorrichtung 61 ist hier gleichzeitig als distales Hüllenteil ausgestaltet, das zusammen mit dem proximalen Hüllenteil 70 die Hülle 60 bildet. Unabhängig davon, ob die Bildübertragungsvorrichtung 11 auch ohne die Sichtfeldvorrichtung 61 als Endoskop einsetzbar ist, bilden die Bildübertragungsvorrichtung 11 und die Sichtfeldvorrichtung 61 zusammen ein Endoskop 10.

Die Sichtfeldvorrichtung 61 umfasst einen Schaft 62 mit einem Beobachtungsfenster 63 und einem Beleuchtungsfenster 64 am distalen Ende. Das Beobachtungsfenster 63 und das Beleuchtungsfenster 64 sind in Anordnung, Größe und Form an das Beobachtungsfenster 24 bzw. das Beleuchtungsfenster 25 am distalen Ende 12 des Schafts 20 der Bildübertragungsvorrichtung 11 angepasst. Das Beobachtungsfenster 63 und das Beleuchtungsfenster 64 sind voneinander optisch getrennt, beispielsweise durch einen Rahmen 65 aus Metall oder aus einem anderen nicht-transparentem Material um das Beobachtungsfenster 63. Alternativ ist ein einziges Fenster vorgesehen, das die Funktionen des Beobachtungsfensters 63 und des oder der Beleuchtungsfenster 64 übernimmt. In diesem Fall kann es sinnvoll oder erforderlich sein, Streulicht zu mindern, beispielsweise durch eine Schwärzung des Fensters an einer Grenze zwischen den beiden Bereichen, die die Funktionen des Beleuchtungsfensters 64 und des Beobachtungsfensters 63 übernehmen. An der Sichtfeldvorrichtung 61 sind O-Ringe 66, 67 und ein Gewinde 68 vorgesehen, deren Funktion weiter unten beschrieben wird.

Am proximalen Hüllenteil 70 ist eine Hülse 71 vorgesehen, die über eine axiale Fixierung 72 rotierbar mit dem proximalen Hüllenteil 70 verbunden ist. Die axiale Fixierung 72 umfasst beispielsweise einen offenen oder geschlossenen Ring, der in ringförmige Nuten am proximalen Hüllenteil 70 und an der Hülse 71 eingreift. Die axiale Fixierung 72 erlaubt eine Rotation der Hülse 71 relativ zum proximalen Hüllenteil 70, verhindert jedoch eine relative axiale Verschiebung. Ferner umfasst die Hülle 71 einen O-Ring 73 zur fluiddichten Abdichtung zwischen dem proximalen Hüllenteil 70 und der Hülse 71.

Das proximale Hüllenteil 70 umfasst am proximalen Ende eine Öffnung 74 zur Aufnahme des proximalen Endes des Sensorträgers 43 und des Steckverbinders 44. Ferner kann das proximale Ende des proximalen Hüllenteils 70 ausgebildet sein, um eine fluiddichte Verbindung mit einem Mantel 75 des Datenkabels 53 oder mit einem Mantel 75 für das Datenkabel 53 auszubilden. Ferner weist das proximale Hüllenteil 70 eine Öffnung 76 oder ein Fenster auf, durch die das Lichtwellenleiterkabel 58 mit den Lichtewellenleitern 48 optisch gekoppelt werden kann.

An der Hülse 71 ist ein Gegengewinde 78 zum Gewinde 68 an der Sichtfeldvorrichtung 61 ausgebildet. Das Gewinde 68 an der Sichtfeldvorrichtung 61 und das Gegengewinde 78 an der Hülse 71 können miteinander verschraubt werden, um eine lösbare mechanische Verbindung zwischen der Sichtfeldvorrichtung 61 und dem proximalen Hüllenteil 70 über die Hülse 71 und die axiale Fixierung 72 zu schaffen. Über den wechselseitigen Eingriff des Gewindes 68 an der Sichtfeldvorrichtung 61 und des Gegengewindes 78 an der Hülse 71 wird ferner eine relative Rotation der Hülse 71 gegenüber der Sichtfeldvorrichtung 61 in eine axiale Verschiebung derselben und damit eine relative axiale Verschiebung der Sichtfeldvorrichtung 61 und des proximalen Hüllenteils 70 umgesetzt.

Ferner umfasst das proximale Hüllenteil 70 Tasten 79, die den Magnetsensoren 39 gegenüber angeordnet sind und Magnete enthalten. Die Tasten 79 sind so ausgestaltet, dass ein Druck bzw. eine Kraft auf eine Taste 79 eine Bewegung eines darin angeordneten Magneten bewirkt, die von dem gegenüberliegenden Magnetsensor 39 erfasst wird. Die Tasten weisen beispielsweise ein elastisches Material auf und sind fluiddicht und insbesondere steril dichtend ausgebildet. Alternativ zu den Tasten 79 und den Magnetsensoren 39 sind andere Einrichtungen als Benutzerschnittstellen vorgesehen.

Proximal des Beobachtungsfensters 63 ist ein Prisma 87 im Beobachtungsstrahlengang an der Sichtfeldvorrichtung 61 angeordnet. Proximal des oder der Beleuchtungsfenster 64 sind ein bzw. mehrere Prismen 88 im Beleuchtungsstrahlengang angeordnet. Die Prismen 87, 88 können mit dem Beobachtungsfenster 63 bzw. dem Beleuchtungsfenster 64 jeweils einstückig ausgebildet sein. Die Prismen 87, 88 lenken Licht durch Brechung und/oder durch Reflexion an Oberflächen (insbesondere durch Totalreflexion) um, ändern also dessen Richtung. Damit beeinflussen die Prismen 87, 88 das Sichtfeld bzw. den erfassten Raumbereich des aus Bildübertragungsvorrichtung 11 und Sichtfeldvorrichtung bzw. Hülle 60 gebildeten Endoskops 10. Wie nachfolgend anhand der Figuren 3 und 4 näher beschrieben wird, sind so mit ein und derselben Bildübertragungsvorrichtung 11 bei Kombinationen mit unterschiedlichen Sichtfeldvorrichtungen 61 unterschiedliche Sichtfelder realisierbar, die sich hinsichtlich ihrer Richtung und ihrer Größe unterscheiden.

Die Hülle 60 aus der Sichtfeldvorrichtung 61 und dem proximalen Hüllenteil 70 mit der Hülse 71 ist so ausgebildet, dass ihre innere Oberfläche an der äußeren Oberfläche der Bildübertragungsvorrichtung 11 anliegt oder von dieser nur einen geringen Abstand aufweist. Ferner sind die Sichtfeldvorrichtung 61, das proximale Hüllenteil 70 und das Endoskop 11 so ausgebildet, dass eine relative axiale Verschiebung der Sichtfeldvorrichtung 61 und des proximalen Hüllenteils 70 eine entsprechende relative axiale Verschiebung des distalen Gehäuseteils 31 und des proximalen Gehäuseteils 32 der Handhabungseinrichtung 30 der Bildübertragungsvorrichtung 11 bewirkt. Eine relative axiale Verschiebung der Sichtfeldvorrichtung 61 und des Hüllenteils 70 - beispielsweise bewirkt durch eine relative Rotation der Hülse 71 gegenüber der Sichtfeldvorrichtung 61 - kann damit beispielsweise eine Verstellung des Fokus bzw. der Anordnung des lichtempfindlichen Sensors 41 relativ zum Objektiv 42 bewirken.

Ferner können die Sichtfeldvorrichtung 61 und das proximale Hüllenteil 70 so ausgebildet sein, dass eine Rotation der Sichtfeldvorrichtung 61 auf das distale Gehäuseteil 31 der Handhabungseinrichtung 30 und den Schaft 20 übertragen wird und/oder dass eine Rotation des proximalen Hüllenteils 70 auf das proximale Gehäuseteil 32 der Handhabungseinrichtung 30 übertragen wird. Zu diesem Zweck weisen beispielsweise die Sichtfeldvorrichtung 61, das proximale Hüllenteil 70 und die Gehäuseteile 31, 32 der Handhabungseinrichtung 30 Nuten und Stege oder andere starre oder elastisch rastende ineinander greifende Details auf, die in den Figuren nicht dargestellt sind. Mit einer Rotation des proximalen Hüllenteils 70 einerseits gegenüber der Sichtfeldvorrichtung 61 und der Hülse 71 andererseits kann damit beispielsweise eine Blickrichtung des Endoskops 10 verändert werden.

Die O-Ringe 66, 67, 73 an der Sichtfeldvorrichtung 61, am proximalen Hüllenteil 70 und an der Hülse 71 und der O-Ring 47 an der Bildübertragungsvorrichtung 11 schaffen eine fluiddichte Abdichtung der Hülle 60. Insbesondere verhindern die O-Ringe 66, 67, 73, 47 ein Eindringen von nicht-sterilem, flüssigem, gasförmigem oder festem Material in die Hülle 60 oder ein Austreten von nicht-sterilem Material aus der Hülle 60. Nach jedem Einsatz des Endoskops 10 mit der Bildübertragungsvorrichtung 11 und der Hülle 60 bei einem medizinischen Eingriff muss deshalb lediglich die Hülle 60 sterilisiert, insbesondere autoklaviert, werden. Die Bildübertragungsvorrichtung 11 selbst muss der thermischen und mechanischen Belastung des Autoklavierens nicht ausgesetzt werden. Diese Funktionalität der Hülle 60 aus Sichtfeldvorrichtung 61 und proximalem Hüllenteil 70 ist eine optionale Funktionalität.

Der bereits bei der oben anhand der Figur 2 dargestellten Hülle zur optischen Isolierung das Beobachtungsfenster 63 umgebende Rahmen 65 ist hier rohrförmig erweitert. Der proximale Rand des Rahmens 65 ist ausgebildet, um bei in die Hülle 60 eingesetzter Bildübertragungsvorrichtung 11 optisch dicht am distalen Rand des Innenrohrs 21 des Schafts 20 der Bildübertragungsvorrichtung 11 anzuliegen und die Einkopplung von Beleuchtungslicht in den Beobachtungsstrahlengang zu verhindern oder zu vermindern.

Bei der oben anhand der Figur 1 dargestellten Bildübertragungsvorrichtung und der oben anhand der Figur 2 dargestellten Hülle sind die Blickrichtung und das Sichtfeld bereits durch die Bildübertragungsvorrichtung vorgegeben. Bei der in Figur 3 gezeigten Bildübertragungsvorrichtung 11 und der in Figur 4 gezeigten Hülle 60 werden eine von der Längsachse der Bildübertragungsvorrichtung 11 abweichende Blickrichtung und ein zu dieser Längsachse nicht symmetrisches Sichtfeld erst durch die Hülle 60, insbesondere die Prismen 87, 88 erzeugt. Materialien und Geometrien der Prismen 87, 88 bestimmen den Winkel zwischen der Blickrichtung und der Längsachse und die Lage des Sichtfelds bzw. des über die Lichtwellenleiter 48 und durch die Beleuchtungsfenster 25, 64 beleuchteten und von dem lichtempfindlichen Sensor 41 durch das Objektiv 42, die Beobachtungsfenster 24, 63 und das Prisma 87 erfassten Raumbereichs.

Wenn die Sichtfeldvorrichtung 61 mit dem Beobachtungsfenster 63, dem oder den Beleuchtungsfenstern 64 und den Prismen 87, 88 relativ zur Bildübertragungsvorrichtung 11 um deren Längsachse rotierbar ist, kann durch Rotation der Sichtfeldvorrichtung 61 und gleichzeitiges Festhalten der Bildübertragungsvorrichtung 11 (beispielsweise über den proximalen Hüllenteil 70) die Blickrichtung des Endoskops 10 auf einem Kegelmantel variiert werden, ohne dass das erfasste Bild dabei kippt.

Die Figuren 3 und 4 zeigen schematische Darstellungen der alternativen Kombination einer Bildübertragungsvorrichtung 11 mit verschiedenen Sichtfeldvorrichtungen 61 zur Erzielung verschiedener Sichtfelder. Figur 3 zeigt verschiedene Sichtfeldvorrichtungen 61, die in Kombination mit der Bildübertragungsvorrichtung 11 unterschiedliche Blickrichtungen 81, 82, 83, 84 mit einem Winkel zur Längsachse der Bildübertragungsvorrichtung 11 und der Sichtfeldvorrichtung 61 von ca. 90°, ca. 60°, ca. 30° und ca. 0° realisieren. Auch Winkel von 15°, 45°, 75° oder andere Winkel oder ein innerhalb eines Winkelintervalls beliebig einstellbarer Winkel sind mit entsprechenden Sichtfeldvorrichtungen realisierbar.

Figur 4 zeigt verschiedene Sichtfeldvorrichtungen 61, die in Kombination mit einer Bildübertragungsvorrichtung 11 bei im Wesentlichen gleicher Blickrichtung 83 unterschiedlich große Sichtfelder bzw. unterschiedlich große Winkel zwischen gegenüberliegenden Grenzen 85 der Sichtfelder realisieren. Diese Kombinierbarkeit gilt auch für die nachfolgend anhand der Figuren 5 bis 10 beschriebenen Bildübertragungsvorrichtungen und Sichtfeldvorrichtungen.

Figur 5 zeigt eine schematische Darstellung einer Bildübertragungsvorrichtung 11, ähnlich der oben anhand der Figur 1 dargestellten Bildübertragungsvorrichtung. Ähnlich der oben anhand der Figur 1 dargestellten Bildübertragungsvorrichtung sind bei der in Figur 5 gezeigten Bildübertragungsvorrichtung 11 das Objektiv 42 und das Sichtfeld symmetrisch und die Blickrichtung parallel zur Längsachse der Bildübertragungsvorrichtung 11 bzw. von dessen Schaft 20. Das Objektiv 42 ist optional.

Abweichend von der oben anhand der Figur 1 dargestellten Bildübertragungsvorrichtungen weist die in Figur 5 gezeigte Bildübertragungsvorrichtung 11 keine Lichtwellenleiter oder andere Einrichtungen zur Übertragung von Beleuchtungslicht zum distalen Ende 12 oder für eine andersartige Beleuchtung eines beobachteten Gegenstands auf. Der Schaft 20 der Bildübertragungsvorrichtung 11 kann dadurch einen deutlich geringeren Querschnitt aufweisen.

Figur 6 zeigte eine schematische Darstellung einer Hülle 60 aus einer Sichtfeldvorrichtung 61 und einem proximalen Hüllenteil 70 für die oben anhand der Figur 5 dargestellte Bildübertragungsvorrichtung 11. Die in Figur 6 gezeigte Hülle 60 und insbesondere die Sichtfeldvorrichtung 61 ähneln in einigen Eigenschaften und Merkmalen den oben anhand der Figur 2 dargestellten Hüllen 60 bzw. Sichtfeldvorrichtung 61. Die in Figur 6 dargestellte Sichtfeldvorrichtung 61 unterscheidet sich von den oben anhand der Figur 2 dargestellten Sichtfeldvorrichtung unter anderem durch ein Führungsrohr 89 im Schaft 62. Das Lumen des Führungsrohrs 89 bildet einen Kanal 90, der sich entlang des Schafts 62 der Sichtfeldvorrichtung 61 vom proximalen Ende bis zum distalen Ende des Schafts 62 erstreckt. Der Querschnitt des Kanals 90 ist an den Querschnitt des Schafts 20 der Bildübertragungsvorrichtung 11 angepasst, so dass der Schaft 20 der Bildübertragungsvorrichtung 11 mit geringem Spiel und geringer Reibung im Kanal 90 um seine Längsachse rotiert und parallel zur Längsachse verschoben werden kann.

Proximal des Beobachtungsfensters 63 ist ein Prisma 87 angeordnet, ähnlich wie bei der oben anhand der Figur 2 dargestellten Hülle. Material und Geometrie des Prismas 87 bewirken, dass das ausgeleuchtete und von der Bildübertragungsvorrichtung 11 erfasste Sichtfeld zur Längsachse der Bildübertragungsvorrichtung 11 asymmetrisch bzw. die Blickrichtung nicht-parallel zu dieser Längsachse ist. Ähnlich wie oben anhand der Figuren 3 und 4 dargestellt, sind mit ein und derselben Bildübertragungsvorrichtung 11 bei Kombinationen mit unterschiedlichen Sichtfeldvorrichtungen 61 unterschiedliche Sichtfelder realisierbar, die sich hinsichtlich ihrer Richtung und ihrer Größe unterscheiden.

Außerhalb des Führungsrohrs 89 aber innerhalb des Schafts 62 verlaufen Lichtwellenleiter 91 von einer Öffnung bzw. einem Anschluss 76 am proximalen Ende der Hülle 60 bis zu einem ringförmigen oder mehreren Beleuchtungsfenstern 64 am distalen Ende der Sichtfeldvorrichtung 61. Anstelle des oder der als transparente Bauteile eingesetzten Beleuchtungsfenster 64 können die distalen Enden der Lichtwellenleiter 91 am distalen Ende des Schafts 62 der Sichtfeldvorrichtung 61 lediglich mittels eines (optional transparenten) Kitts eingekittet sein. Durch Überschleifen und Polieren der distalen Stirnfläche des Schafts 62 mit den Enden der Lichtwellenleiter 48 und dem Kitt ist eine optisch hochwertige Lichtaustrittsfläche erzielbar.

Über ein in Figur 6 nicht dargestelltes Lichtwellenleiterkabel, das mit der Öffnung bzw. dem Anschluss 76 verbunden ist, kann Licht von einer externen Lichtquelle über die Lichtwellenleiter 91 zum distalen Ende der Sichtfeldvorrichtung 61 geleitet werden, um dort einen Gegenstand außerhalb der Hülle 60 zu beleuchten. Die Anordnung des oder der Beleuchtungsfenster 64 und der distalen Enden der Lichtwellenleiter 91 bewirken, dass das ausgeleuchtete Sichtfeld das von der Bildübertragungsvorrichtung 11 erfasste Sichtfeld umfasst oder ihm im Wesentlichen entspricht.

Figur 7 zeigt eine schematische Darstellung einer Bildübertragungsvorrichtung 11 ähnlich der oben anhand der Figur 5 dargestellten Bildübertragungsvorrichtung. Abweichend von der oben anhand der Figur 5 dargestellten Bildübertragungsvorrichtung weist die in Figur 7 gezeigte Bildübertragungsvorrichtung 11 keine Einrichtung zur Veränderung des Fokus bzw. zur Variation des Abstands zwischen dem lichtempfindlichen Sensor 41 und dem Objektiv 42 auf. Das Objektiv 42 ist optional.

Figur 8 zeigt eine schematische Darstellung einer Hülle 60 aus einer Sichtfeldvorrichtung 61 und einem proximalen Hüllenteil 70 für die oben anhand der Figur 7 dargestellte Bildübertragungsvorrichtung 11. Die in Figur 8 gezeigte Sichtfeldvorrichtung 61 ähnelt der oben anhand der Figur 6 dargestellten Sichtfeldvorrichtung weitgehend. Die in Figur 8 gezeigte Sichtfeldvorrichtung 61 weist am distalen Ende eine Linse 92 im Beobachtungsstrahlengang auf, die bei den oben anhand der Figuren 2 und 6 dargestellten Hüllen nicht dargestellt ist aber optional ebenfalls vorgesehen sein kann.

Wie bereits oben anhand der Figur 2 dargestellt, kann auch bei den in den Figuren 6 und 8 dargestellten Sichtfeldvorrichtungen durch eine Rotation der Hülse 71 relativ zur Sichtfeldvorrichtung 61 eine relative axiale Verschiebung der Sichtfeldvorrichtung 61 und des proximalen Hüllenteils 70 bewirkt werden. Bei der in Figur 8 dargestellten Kombination der Hülle 60 mit der Bildübertragungsvorrichtung 11 bewirkt eine so oder anders hervorgerufene relative axiale Verschiebung der Sichtfeldvorrichtung 61 und des proximalen Hüllenteils 70 eine relative axiale Verschiebung der Bildübertragungsvorrichtung 11 und der Sichtfeldvorrichtung 61. Es resultiert insbesondere eine relative axiale Verschiebung des lichtempfindlichen Sensors 41 und ggf. des Objektivs 42 einerseits und des Objektivs 92 am distalen Ende der Sichtfeldvorrichtung 61 andererseits. Dies bewirkt eine Veränderung der Fokussierung bzw. eine Verschiebung der vom Objektiv 92 an der Sichtfeldvorrichtung 61 und ggf. vom Objektiv 42 der Bildübertragungsvorrichtung 11 scharf auf den lichtempfindlichen Sensor 41 abgebildeten Ebene.

Ähnlich wie bei den oben anhand der Figuren 1 und 5 dargestellten Bildübertragungsvorrichtungen und den oben anhand der Figuren 2 und 6 dargestellten Sichtfeldvorrichtungen kann auch bei der anhand der Figur 7 dargestellten Bildübertragungsvorrichtung 11 und der anhand der Figur 8 dargestellten Sichtfeldvorrichtung 61 eine relative Rotierbarkeit der Bildübertragungsvorrichtung 11 und der Sichtfeldvorrichtung 61 vorgesehen sein. Dadurch kann, wie oben dargestellt, eine Rotation der Blickrichtung des Endoskops 10 auf einen Kegelmantel ohne Kippen des Bilds ermöglicht werden.

Figur 9 zeigt eine schematische Darstellung einer Bildübertragungsvorrichtung 11 ähnlich der oben anhand der Figur 7 dargestellten Bildübertragungsvorrichtung. Die in Figur 9 gezeigte Bildübertragungsvorrichtung 11 unterscheidet sich von der oben anhand der Figur 7 dargestellten Bildübertragungsvorrichtung durch eine etwas andere Ausgestaltung der Handhabungseinrichtung 30 mit einer proximal-stirnseitigen Nut, in der O-Ringe 94 angeordnet sind. Ferner ist im Inneren der Handhabungseinrichtung 30 eine Platine 95 mit einer analogen und/oder digitalen elektronischen Schaltung gezeigt, mit der die Magnetfeldsensoren 39 und der lichtempfindliche Sensor 41 gekoppelt sind, und die beispielsweise zur Signalaufbereitung oder Signalverarbeitung ausgebildet ist.

Figur 10 zeigt eine schematische Darstellung einer Sichtfeldvorrichtung 61 ähnlich der oben anhand der Figur 8 dargestellten Sichtfeldvorrichtung. Die in Figur 10 gezeigte Sichtfeldvorrichtung 61 ist jedoch zur Verwendung ohne ein proximales Hüllenteil ausgebildet und vorgesehen. Stattdessen ist die Sichtfeldvorrichtung 61 ausgebildet, um in die erwähnte proximal-stirnseitige Nut der Handhabungseinrichtung 30 der Bildübertragungsvorrichtung 11 einzugreifen. O-Ringe 94 an der Handhabungseinrichtung 30 und an der Sichtfeldvorrichtung 61 sind zur fluiddichten Abdichtung der Verbindung zwischen dem proximalen Ende der Sichtfeldvorrichtung 61 und der Handhabungseinrichtung 30 der Bildübertragungsvorrichtung 11 ausgebildet und angeordnet.

Die in Figur 10 gezeigte Sichtfeldvorrichtung 61 weist eine exzentrische Anordnung des Führungsrohrs 89 im Schaft 62 auf. Es resultiert unter anderem eine entsprechend asymmetrische Anordnung des Beobachtungsfensters 63 und des Beleuchtungsfensters 64 sowie der distalen Enden der Lichtwellenleiter 91. Proximal des Beobachtungsfensters 63 weist die Sichtfeldvorrichtung 61 ein Prisma 87 auf, das durch das Beobachtungsfenster 63 einfallendes Licht durch Totalreflexion an Oberflächen des Prismas 87 zum Objektiv 92 und über dieses zum lichtempfindlichen Sensor 41 der Bildübertragungsvorrichtung 11 umlenkt.

Auch bei der in Figur 10 gezeigten Sichtfeldvorrichtung können anstelle der Verwendung von als transparente Bauteile eingesetzten Beleuchtungsfenster 64 die distalen Enden der Lichtwellenleiter 91 am distalen Ende des Schafts 62 der Sichtfeldvorrichtung 61 lediglich mittels eines (optional transparenten) Kitts eingekittet sein. Durch Überschleifen und Polieren der distalen Stirnfläche des Schafts 62 mit den Enden der Lichtwellenleiter 48 und dem Kitt ist eine optisch hochwertige Lichtaustrittsfläche erzielbar.

Am proximalen Ende des Objektivs 92 ist ein Deckglas 93 angeordnet, das die optische Einrichtung mit Prisma 87 und Objektiv 92 am distalen Ende der Sichtfeldvorrichtung 61 zum Kanal 90 hin fluiddicht abdichtet. Die optische Einrichtung mit dem Prisma 87 und dem Objektiv 92 ist somit durch das Beobachtungsfenster 63 nach distal und durch das Deckglas 93 nach proximal vollständig fluiddicht gekapselt. Die Sichtfeldvorrichtung 61 kann deshalb uneingeschränkt autoklaviert werden, ohne die optischen Eigenschaften der optischen Einrichtung am distalen Ende zu beeinträchtigen.

Ähnlich wie die oben anhand der Figur 10 dargestellte Sichtfeldvorrichtung 61 können auch die oben anhand der Figuren 2, 6 und 8 dargestellten Sichtfeldvorrichtungen abweichend von der obigen Darstellung für eine Verwendung ohne ein proximales Hüllenteil 70 ausgebildet sein. Um trotzdem eine sterile Ummantelung oder Umhüllung der Bildübertragungsvorrichtung 11 zu ermöglichen, kann ein Steriltuch verwendet werden. Dieses Steriltuch kann mit der Sichtfeldvorrichtung 61 proximal verbunden sein.

Jede der anhand der Figuren 2, 6, 8 und 10 dargestellten Sichtfeldvorrichtungen kann für unterschiedliche Sichtfelder, insbesondere für unterschiedliche Blickrichtungen und unterschiedliche Größen der Sichtfelder ausgebildet sein. Wie bereits erwähnt können auch die anhand der Figuren 5 bis 10 dargestellten Bildübertragungsvorrichtungen ähnlich wie oben anhand der Figuren 3 und 4 dargestellt mit unterschiedlichen Sichtfeldvorrichtungen zu Endoskopen mit unterschiedlichen Sichtfeldern kombiniert werden.

Jede der oben anhand der Figuren 1, 3, 5 und 7 dargestellten Bildübertragungsvorrichtungen kann in der Handhabungseinrichtung am proximalen Ende eine oder mehrere Platinen mit einer oder mehreren analogen oder digitalen elektronischen Schaltungen zur Signalaufbereitung oder Signalverarbeitung aufweisen, wie dies oben anhand der Figur 9 dargestellt ist.

Wie bereits oben anhand der Figuren 1 und 2 beschrieben, können auch bei den oben anhand der Figuren 6, 8 und 10 dargestellten Sichtfeldvorrichtungen anstelle der separaten Beobachtungsfenster und Beleuchtungsfenster jeweils ein gemeinsames Fenster bzw. ein einstückiges transparentes Bauteil vorgesehen sein. In diesem Fall tritt durch dieses eine Fenster Licht zur Beleuchtung eines Objekts in einer Richtung und vom beleuchteten Objekt reflektiertes oder gestreutes Licht in umgekehrter Richtung hindurch.

Figur 11 zeigt eine Variante der oben anhand der Figur 5 dargestellten Bildübertragungsvorrichtung 11. Die in Figur 11 dargestellte Bildübertragungsvorrichtung 11 weist anstelle eines Sensorträgers 43 einen Optikträger 96 auf. Der Optikträger 96 wird ähnlich wie der Sensorträger der oben dargestellten Bildübertragungsvorrichtungen von einer Feder nach proximal gedrückt und stützt sich am proximalen Gehäuseteil 32 der Handhabungseinrichtung 30 ab. Im Schaft 20 der Bildübertragungsvorrichtung 11 ist der Optikträger 96 rohrförmig ausgebildet und enthält eine Anordnung von Stablinsen 97. Am proximalen Ende der Anordnung von Stablinsen 97 in der Handhabungseinrichtung 30 trägt der Optikträger eine Prismeneinrichtung 98 mit mehreren, insbesondere drei, lichtempfindlichen Sensoren 41. Die drei lichtempfindlichen Sensoren 41 sind mit einer elektronischen Schaltung an einer Platine 95 gekoppelt.

Anstelle der in Figur 11 dargestellten Anordnung von drei Sensoren 41 an einer Prismeneinrichtung 98 kann eine andere Anzahl von Sensoren oder lediglich ein Sensor, der für Licht mit verschiedenen Wellenlängen empfindlich ist, vorgesehen sein. Als Sensoren sind beispielsweise CMOS- oder CCD-Sensoren verwendbar. Ferner kann anstelle von einem oder mehreren Sensoren ein Okular zur unmittelbaren Beobachtung durch das menschliche Auge oder eine Kupplung zur Ankopplung einer Kamera am proximalen Ende der Bildübertragungseinrichtung 11 vorgesehen sein.

Das Objektiv 42 ist am distalen Ende des Schafts 20 der Bildübertragungsvorrichtung 30 befestigt und weist damit eine unveränderliche Position relativ zum distalen Gehäuseteil 31 der Handhabungseinrichtung 30 auf. Die Stablinsen 97, die Prismenanordnung 98 und die lichtempfindlichen Sensoren 41 sind am Optikträger 81 befestigt, der sich am proximalen Gehäuseteil 32 der Handhabungseinrichtung 30 abstützt. Die Stablinse 97, die Prismenanordnung 98 und die lichtempfindlichen Sensoren 41 weisen deshalb eine unveränderliche Position relativ zum proximalen Gehäuseteil 32 der Handhabungseinrichtung 30 auf. Eine relative Verschiebung des proximalen Gehäuseteils 32 und des distalen Gehäuseteils 31 der Handhabungseinrichtung 30 bewirkt somit eine relative Verschiebung des Objektivs 42 einerseits gegenüber der Anordnung der Stablinsen 97, der Prismeneinrichtung 98 und den lichtempfindlichen Sensoren 41 andererseits.

Licht, das durch das Beobachtungsfenster 24 in die Bildübertragungsvorrichtung 11 eintritt, wird von dem Objektiv 42 und der Anordnung von Stablinsen 97 zum proximalen Ende 13 der Bildübertragungsvorrichtung 13 übertragen, von der Prismeneinrichtung 98 in mehrere Wellenlängenbereiche aufgespaltet und abhängig von der Wellenlänge auf einen (oder mehrere) der Sensoren 41 abgebildet. Die lichtempfindlichen Sensoren wandeln das Licht in elektrische Signale, die von der elektronischen Schaltung an der Platine 95 aufbereitet und verarbeitet, insbesondere verstärkt und digitalisiert werden.

Figur 12 zeigt eine Hülle 60 mit einer Sichtfeldvorrichtung 61, die ein distales Hüllenteil bildet, und einem proximalen Hüllenteil 70, ähnlich der oben anhand der Figur 8 dargestellten Sichtfeldvorrichtung. Die in Figur 12 dargestellte Sichtfeldvorrichtung sei jedoch so ausgebildet, dass der distale Gehäuseteil 31 der Handhabungseinrichtung 30 und der Schaft 20 gegenüber der Sichtfeldvorrichtung 61 verschiebbar sind. Somit sind sowohl das Objektiv 42 der Bildübertragungsvorrichtung 11 einerseits als auch die Anordnung der Stablinsen 97, die Prismeneinrichtung 98 und die lichtempfindlichen Sensoren 41 andererseits unabhängig von einander relativ zu dem Objektiv 92 am distalen Ende der Sichtfeldvorrichtung 61 bewegbar. Damit ist bei entsprechender Ausbildung des Objektivs 92 am distalen Ende der Sichtfeldvorrichtung 61, des Objektivs 42 am distalen Ende der Bildübertragungsvorrichtung 11, der Anordnung der Stablinsen 97, der Prismeneinrichtung 98 und der lichtempfindlichen Sensoren 41 sowohl eine Verstellung der Brennweite bzw. eine Verstellung der Größe des Sichtfelds als auch eine Fokussierung bzw. eine Verstellung der scharf auf die lichtempfindlichen Sensoren 41 abgebildeten Fläche möglich.

Die beschriebene Verstellung der Brennweite und des Fokus ist nicht nur mit der oben anhand der Figur 11 dargestellten Bildübertragungsvorrichtung 11 sondern beispielsweise auch bei Kombination der oben anhand der Figur 5 dargestellten Bildübertragungsvorrichtung mit einer der oben anhand der Figuren 8 und 12 dargestellten Sichtfeldvorrichtungen möglich. Ferner ist eine Verstellung der Brennweite und des Fokus bei Kombination der oben anhand der Figur 1 dargestellten Bildübertragungsvorrichtung mit der oben anhand der Figur 2 dargestellten Sichtfeldvorrichtung möglich, wenn diese abweichend von der Darstellung in Figur 2 am distalen Ende ein Objektiv bzw. eine Linse aufweist.

Zur beschriebenen Verstellung der Brennweite und des Fokus ist, wie erwähnt, eine unabhängige Verschiebung des distalen Gehäuseteils 31 der Handhabungseinrichtung 30 der Bildübertragungsvorrichtung 11 mit dem Schaft 20 einerseits und des proximalen Gehäuseteils der Handhabungseinrichtung 30 andererseits gegenüber der Sichtfeldvorrichtung 61 erforderlich. Dazu sind die Gehäuseteile 31, 32 der Handhabungseinrichtung, die Sichtfeldvorrichtung 61 und das proximale Hüllenteil 70 beispielsweise abweichend von der Darstellung in Figur 12 so ausgebildet, dass eine axiale Verschiebung des proximalen Hüllenteil 70 gegenüber der Sichtfeldvorrichtung 61 eine Verstellung der Brennweite bewirkt und eine Rotation des proximalen Hüllenteil 70 gegenüber der Sichtfeldvorrichtung 61 eine Verstellung des Fokus bewirkt.

Bei Sichtfeldvorrichtungen 61 und Bildübertragungsvorrichtungen 11, wie sie oben anhand der Figuren 1 bis 12 dargestellt sind, kann - wie bereits erwähnt - durch Austausch der Sichtfeldvorrichtung 61 das Sichtfeld verändert werden. Wenn die Blickrichtung nicht parallel zur Längsachse der Sichtfeldvorrichtung 61 und der Bildübertragungsvorrichtung 11 ist, kann durch Rotieren der Sichtfeldvorrichtung 61 um die Längsachse die Blickrichtung auf einem Kegelmantel rotiert werden. Bei einigen Ausführungsformen können Sichtfeldvorrichtung 61 und Bildübertragungsvorrichtung 11 unabhängig von einander rotiert werden. Durch Drehen der Bildübertragungsvorrichtung 11 kann in diesem Fall das von einer Kamera erfasste Bild gedreht, insbesondere aufgerichtet werden. Die Kamera kann dabei am distalen oder am proximalen Ende der Bildübertragungsvorrichtung 11 angeordnet oder mit dem proximalen Ende der Bildübertragungsvorrichtung gekoppelt sein. Beispielsweise bei den oben anhand der Figuren 1 und 2, 5 und 6, 11 und 12 dargestellten Ausführungsformen kann das erfasste Bild statt durch eine Rotation der gesamten Bildübertragungsvorrichtung 11 durch eine Rotation lediglich des Sensorträgers 43 bzw. des Optikträgers 96 oder der Prismeneinrichtung 98 rotiert werden.

Alternativ oder gleichzeitig können die Sichtfeldvorrichtung 61 und die Bildübertragungsvorrichtung 11 so ausgebildet sein, dass eine Rotation der beiden relativ zu einander eine axiale Verschiebung von einer oder mehreren Linsen bewirkt. Dazu sind beispielsweise ein Gewinde, eine helikale Nut oder ein helikaler Steg an der Sichtfeldvorrichtung 61 und eine axiale Nut oder ein axialer Steg an der Bildübertragungsvorrichtung 11 vorgesehen. Eine Fassung der Linse oder der Linsen ist in Eingriff sowohl mit dem Gewinde, der helikalen Nut bzw. dem helikalen Steg an der Sichtfeldvorrichtung 61 als auch mit der axialen Nut bzw. dem axialen Steg an der Bildübertragungsvorrichtung 11. Alternativ sind das Gewinde, die helikale Nut bzw. der helikale Steg an der Bildübertragungsvorrichtung 11 und die axiale Nut bzw. der axiale Steg an der Sichtfeldvorrichtung 1 vorgesehen. Auch zwei Gewinde oder helikale Strukturen mit gegensätzlicher Windungsrichtung sind möglich.

In den beschriebenen Fällen kann eine relative Rotation der Sichtfeldeinrichtung 61 und der Bildübertragungseinrichtung 11 eine axiale Bewegung der Linse bzw. der Linsen bewirken. Durch die axiale Bewegung der Linse bzw. der Linsen können der Fokus und/oder die Brennweite und damit die Größe des Sichtfelds verändert werden.

Alternativ oder gleichzeitig kann - wie teilweise bereits beschrieben - eine einfache axiale Relativbewegung von Sichtfeldvorrichtung 61 und Bildübertragungsvorrichtung 11 eine Veränderung von Fokus und/oder Brennweite bzw. Sichtfeld bewirken. Dazu werden durch die axiale Relativbewegung eine oder mehrere Linsen oder ein Stablinsensystem oder ein lichtempfindlicher Sensor relativ zu einander und/oder zum Objekt verschoben. Die Relativbewegung wird beispielsweise - wie bereits angedeutet - durch eine Rotation der rotierbaren Hülse 71 erzeugt, die über die axiale Fixierung 72 mit dem proximalen Hüllenteil 70 rotierbar aber nicht axial verschiebbar verbunden ist. Ineinander greifende Gewinde 68, 78 an der Sichtfeldvorrichtung 61 bzw. an der rotierbaren Hülse 71 können eine Rotation der rotierbaren Hülse 71 relativ zur Sichtfeldvorrichtung 61 in eine axiale Verschiebung der Sichtfeldvorrichtung 61 relativ zur proximalen Hülle 71 bewirken, wobei die Bildübertragungsvorrichtung 11 mit der proximalen Hülle 71 beispielsweise kraftschlüssig oder formschlüssig verbunden ist.

Figur 13 zeigt ein schematisches Flussdiagramm eines Verfahrens zum Bereitstellen eines Endoskops und zum Autoklavieren einer Sichtfeldvorrichtung. Obwohl dieses Verfahren auch mit Bildübertragungsvorrichtungen - insbesondere Endoskopen - und Sichtfeldvorrichtungen, die sich von den oben anhand der Figuren 1 bis 12 dargestellten unterscheiden, ausführbar ist, werden nachfolgend Bezugszeichen aus den Figuren 1 bis 12 verwendet, um ein Verständnis des Verfahrens zu erleichtern.

Bei einem ersten Schritt 101 wird ein bei einer nachfolgenden Verwendung erforderliches Sichtfeld bestimmt. Bei einem zweiten Schritt 102 wird eine Sichtfeldvorrichtung 61 mit dem bestimmten Sichtfeld 90 ausgewählt. Bei einem dritten Schritt 103 wird eine Bildübertragungsvorrichtung 11 mit der ausgewählten Sichtfeldvorrichtung 61 kombiniert bzw. zusammengeführt, insbesondere in den Kanal 90 der ausgewählten Sichtfeldvorrichtung 61 eingeführt, um ein Endoskop 10 zu bilden.

Der erste Schritt 101, der zweite Schritt 102 und der dritte Schritt 103 bilden ein Verfahren zum Bereitstellen eines Endoskops für eine nachfolgende Verwendung des Endoskops, beispielsweise bei einer medizinischen Untersuchung oder einem medizinischen Eingriff.

Bei einem vierten Schritt 104 wird das mit dem ersten Schritt 101, dem zweiten Schritt 102 und dem dritten Schritt 103 bereitgestellte Endoskop verwendet. Nach der Verwendung des Endoskops 10 wird bei einem fünften Schritt 105 die Bildübertragungsvorrichtung 11 aus der Sichtfeldvorrichtung 61 entnommen.

Nach dem Entnehmen der Bildübertragungsvorrichtung 11 im fünften Schritt 105 wird bei einem sechsten Schritt 106 der Kanal 90 am proximalen Ende fluiddicht verschlossen, beispielsweise mittels eines Dichtstopfens. Bei einem siebten Schritt 107 wird die Sichtfeldvorrichtung 61 mit dem verschlossenen Kanal 90 autoklaviert. Nach dem Autoklavieren wird bei einem achten Schritt 108 der Kanal 90 geöffnet. Der sechste Schritt 106 und der achte Schritt 108 sind nicht erforderlich, wenn die optische Einrichtung am distalen Ende der Sichtfeldvorrichtung beispielsweise wie oben anhand der Figur 10 dargestellt, vollständig gekapselt bzw. sowohl nach distal als auch nach proximal fluiddicht verschlossen ist.

Der sechste Schritt 106, der siebte Schritt 107 und der achte Schritt 108 bilden ein Verfahren zum Autoklavieren der Sichtfeldvorrichtung. Nach dem Autoklavieren der Sichtfeldvorrichtung kann erneut eine Bildübertragungsvorrichtung 11 in die Sichtfeldvorrichtung 61 eingeführt werden, um den dritten Schritt 103 und die nachfolgenden Schritte zu wiederholen.

### Bezugszeichen

- 10: Endoskop
- 11: Bildübertragungsvorrichtung
- 12: distales Ende der Bildübertragungsvorrichtung 11
- 13: proximales Ende der Bildübertragungsvorrichtung 11
- 20: Schaft der Bildübertragungsvorrichtung 11
- 21: Innenrohr des Schafts 20
- 22: Außenrohr des Schafts 20
- 24: Beobachtungsfenster am Schaft 20 der Bildübertragungsvorrichtung 11
- 25: Beleuchtungsfenster am Schaft 20 des der Bildübertragungsvorrichtung 11
- 30: Handhabungseinrichtung der Bildübertragungsvorrichtung 11
- 31: distales Gehäuseteil der Handhabungseinrichtung 30
- 32: proximales Gehäuseteil der Handhabungseinrichtung 30
- 34: Verschraubung des Innenrohr 21 mit dem distalen Gehäuseteil 31
- 35: Kontermutter
- 37: O-Ring zwischen proximalem Gehäuseteil 31 und distalem Gehäuseteil 31
- 39: Magnetsensor
- 41: lichtempfindlicher Sensor
- 42: Objektiv
- 43: Sensorträger
- 44: Steckverbinder am proximalen Ende des Sensorträgers 43
- 46: O-Ring zwischen dem Sensorträger 43 und dem proximalen Gehäuseteil 32
- 47: O-Ring zwischen dem Sensorträger 43 und dem proximalen Hüllenteil 70
- 48: Lichtwellenleiter für Beleuchtungslicht
- 49: Durchführung oder Steckverbinder für Lichtwellenleiter 48
- 53: Signalkabel
- 54: Steckverbinder am Signalkabel 53
- 58: Lichtwellenleiterkabel
- 59: Steckverbinder am Lichtwellenleiterkabel 58
- 60: Hülle
- 61: Sichtfeldvorrichtung und distales Hüllenteil der Hülle 60
- 62: Schaft der Sichtfeldvorrichtung 61 der Hülle 60
- 63: Beobachtungsfenster an der Sichtfeldvorrichtung 61
- 64: Beleuchtungsfenster an der Sichtfeldvorrichtung 61
- 65: Rahmen zwischen Beobachtungsfenster 63 und Beleuchtungsfenster 64
- 66: O-Ring zwischen der Sichtfeldvorrichtung 61 und der Hülse 71
- 67: O-Ring zwischen der Sichtfeldvorrichtung 61 und dem distalen Hüllenteil 70
- 68: Gewinde an der Sichtfeldvorrichtung 61
- 70: proximales Hüllenteil
- 71: rotierbare Hülse am proximalen Hüllenteil 70
- 72: axiale Fixierung
- 73: O-Ring zwischen dem proximalen Hüllenteil 70 und der Hülse 71
- 74: Öffnung im proximalen Hüllenteil 70
- 75: Mantel für Datenkabel 53
- 76: Öffnung im proximalen Hüllenteil 70 für Lichtwellenleiterkabel 58
- 78: Gegengewinde am proximalen Hüllenteil 70
- 79: Taste
- 81: Blickrichtung
- 82: Blickrichtung
- 83: Blickrichtung
- 84: Blickrichtung
- 85: Grenze des Sichtfelds
- 87: Prisma in Beobachtungsstrahlengang
- 88: Prisma in Beleuchtungsstrahlengang
- 89: Führungsrohr im Schaft 62
- 90: Kanal im Führungsrohr 89
- 91: Lichtwellenleiter
- 92: Objektiv in Beobachtungsstrahlengang
- 93: Deckglas zum Kanal 90
- 94: O-Ring zwischen der Handhabungseinrichtung 30 und der Sichtfeldvorrichtung 61
- 95: Platine
- 96: Optikträger
- 97: Stablinse
- 98: Prismeneinrichtung
- 101: erster Schritt
- 102: zweiter Schritt
- 103: dritter Schritt
- 104: vierter Schritt
- 105: fünfter Schritt
- 106: sechster Schritt
- 107: siebter Schritt
- 108: achter Schritt

## Patentansprüche

1. **Hülle (60)** für eine Bildübertragungsvorrichtung (11), mit:
einer **austauschbaren Sichtfeldvorrichtung** (61) mit einem Kanal (90), der das proximale Ende und das distale Ende der Sichtfeldvorrichtung (61) verbindet, zum Aufnehmen der Bildübertragungsvorrichtung (11),
wobei der **Querschnitt** des Kanals der Sichtfeldvorrichtung (61) auf den Querschnitt der Bildübertragungsvorrichtung **abgestimmt** ist, um eine **Rotation** der Bildübertragungsvorrichtung (11) um ihre Längsachse in dem Kanal (90) zu ermöglichen;
einer optischen **Einrichtung** (87, 92) am distalen Ende des Kanals (90), die ausgebildet ist, um zumindest entweder das **Sichtfeld** oder die Fokussierung der Bildübertragungsvorrichtung (11) zu beeinflussen,
wobei die Sichtfeldvorrichtung (61) als ein **austauschbares distales Hüllenteil** ausgebildet ist,
wobei die **Sichtfeldvorrichtung** (61) ausgebildet ist, um mit der **Bildübertragungsvorrichtung** (11) zusammen ein **Endoskop** (10) zu bilden,
**gekennzeichnet durch** ein **proximales Hüllenteil** (70) mit einer Hülse (71), wobei die Hülse (71) an dem proximalen Hüllenteil (70) vorgesehen ist, und wobei die Hülse (71) über eine axiale Fixierung (72) rotierbar mit dem proximalen Hüllenteil (70) verbunden ist,
wobei die Sichtfeldvorrichtung (61) und das proximale Hüllenteil (70) mit der Hülse (71) **ausgebildet** sind, **um** eine lösbare mechanische Verbindung zwischen der Sichtfeldvorrichtung (61) und dem proximalen Hüllenteil (70) über die Hülse (71) und die axiale Fixierung (72) zu schaffen und die Hülle (60) zu bilden.

2. Hülle (60) nach dem vorangehenden Anspruch, bei der die optische Einrichtung (87, 92) ausgebildet ist, um Licht zumindest entweder zu **brechen** oder zu **reflektieren** oder zu **beugen.**

3. Hülle (60) nach einem der vorangehenden Ansprüche, bei der die optische Einrichtung zumindest entweder ein **Prisma** (87) oder einen **Spiegel** oder eine **Linse** (92) umfasst.

4. Hülle (60) nach einem der vorangehenden Ansprüche, ferner mit einer **Justageeinrichtung** (68, 71, 78) zur justierbaren Festlegung der Position einer Bildübertragungsvorrichtung (11) in Längsrichtung in dem Kanal (90).

5. Hülle (60) nach einem der vorangehenden Ansprüche, ferner mit:
einem **Lichtwellenleiter** (91) zur Übertragung von Beleuchtungslicht vom proximalen Ende zum distalen Ende der Sichtfeldvorrichtung (61) zur Beleuchtung eines zu betrachtenden Objekts.

6. Hülle (60) nach einem der vorangehenden Ansprüche, ferner mit:
einer **Lichtaustrittseinrichtung** (64) am distalen Ende der Sichtfeldvorrichtung (61), zum Leiten von Beleuchtungslicht in einen Raumbereich außerhalb der Sichtfeldvorrichtung (61).

7. Hülle (60) nach einem der vorangehenden Ansprüche, bei welcher die **optische Einrichtung** (87, 92) **fluiddicht** nach außen oder nach außen und zum Kanal (90) hin abgedichtet ist.

8. **Endoskop** (10) mit einer Hülle (60) nach einem der Ansprüche 1 bis 7 und einer Bildübertragungseinrichtung (11), wobei die Bildübertragungsvorrichtung (11) in den Kanal (90) der Sichtfeldvorrichtung (61) einsetzbar ist, wobei die Bildübertragungsvorrichtung (11) zumindest entweder eine Einrichtung zum optischen Übertragen eines Bilds von einem distalen Ende zu einem proximalen Ende des Endoskops (10) oder einen lichtempfindlichen Sensor (41) zum Wandeln eines Bilds in ein Bildsignal aufweist.

9. Endoskop (10) nach dem vorangehenden Anspruch, wobei die Bildübertragungsvorrichtung (11) umfasst:
einen **Schaft** (20), der ausgebildet ist, um in den Kanal (90) der Sichtfeldvorrichtung (61) eingesetzt zu werden;
zumindest entweder eine Einrichtung zum optischen **Übertragen** eines Bilds von einem distalen Ende (12) zu einem proximalen Ende (13) des Schafts (20) oder einen lichtempfindlichen Sensor (41) zum Wandeln eines optischen Bilds in ein Bildsignal.

## Claims

1. Sleeve (60) for an image transmission apparatus (11), comprising:
an interchangeable field-of-view apparatus (61) with a channel (90), which connects the proximal end and the distal end of the field-of-view apparatus (61), for receiving the image transmission apparatus (11),
wherein the cross section of the channel of the field-of-view apparatus (61) is matched to the cross section of the image transmission apparatus in order to facilitate a rotation of the image transmission apparatus (11) about its longitudinal axis in the channel (90),
an optical device (87, 92) at the distal end of the channel (90), which is embodied to influence at least either the field of view or the focusing of the image transmission apparatus (11),
wherein the field-of-view apparatus (61) is embodied as an interchangeable distal sleeve part,
wherein the field-of-view apparatus (61) is embodied to form an endoscope (10) together with the image transmission apparatus (11),
**characterized by** a proximal sleeve part (70) with a sheath (71), wherein the sheath (71) is provided at the proximal sleeve part (70) and wherein the sheath (71) is connected in rotatable fashion to the proximal sleeve part (70) by way of an axial fixation device (72),
wherein the field-of-view apparatus (61) and the proximal sleeve part (70) are embodied with the sheath (71) in order to create a detachable mechanical connection between the field-of-view apparatus (61) and the proximal sleeve part (70) via the sheath (71) and the axial fixation device (72) and in order to form the sleeve (60).

2. Sleeve (60) according to the preceding claim, wherein the optical device (87, 92) is embodied to at least either refract or reflect or diffract light.

3. Sleeve (60) according to any one of the preceding claims, wherein the optical device comprises at least either a prism (87) or a mirror or a lens (92) .

4. Sleeve (60) according to any one of the preceding claims, further comprising an adjustment device (68, 71, 78) for adjustably setting the position of an image transmission apparatus (11) in the channel (90) in a longitudinal direction.

5. Sleeve (60) according to any one of the preceding claims, further comprising:
an optical waveguide (91) for transmitting illumination light from the proximal end to the distal end of the field-of-view apparatus (61) for the purposes of illuminating an object to be observed.

6. Sleeve (60) according to any one of the preceding claims, further comprising:
a light outlet device (64) at the distal end of the field-of-view apparatus (61), for guiding illumination light into a spatial region outside of the field-of-view apparatus (61).

7. Sleeve (60) according to any one of the preceding claims, in which the optical device (87, 92) is sealed in fluid-tight fashion to the outside or to the outside and toward the channel (90).

8. Endoscope (10) comprising a sleeve (60) according to any one of Claims 1 to 7 and an image transmission apparatus (11), wherein the image transmission apparatus (11) is insertable into the channel (90) of the field-of-view apparatus (61), wherein the image transmission apparatus (11) has at least either a device for optically transmitting an image from a distal end to a proximal end of the endoscope (10) or a light-sensitive sensor (41) for converting an image into an image signal.

9. Endoscope (10) according to the preceding claim, wherein the image transmission apparatus (11) comprises:
a shaft (20), which is embodied to be inserted into the channel (90) of the field-of-view apparatus (61);
at least either a device for optically transmitting an image from a distal end (12) to a proximal end (13) of the shaft (20) or a light-sensitive sensor (41) for converting an optical image into an image signal.

## Revendications

1. Gaine (60) pour un arrangement de transmission d'images (11), comprenant :
un arrangement à champ visuel (61) remplaçable muni d'un canal (90) qui relie l'extrémité proximale et l'extrémité distale de l'arrangement à champ visuel (61), destiné à accueillir l'arrangement de transmission d'images (11),
la section transversale du canal de l'arrangement à champ visuel (61) étant accordée à la section transversale de l'arrangement de transmission d'images afin de rendre possible une rotation de l'arrangement de transmission d'images (11) autour de son axe longitudinal dans le canal (90) ;
un dispositif optique (87, 92) au niveau de l'extrémité distale du canal (90), qui est configuré pour influencer au moins soit le champ visuel, soit la mise au point de l'arrangement de transmission d'images (11),
l'arrangement à champ visuel (61) étant réalisé sous la forme d'une partie de gaine distale remplaçable,
l'arrangement à champ visuel (61) étant configuré pour, conjointement avec l'arrangement de transmission d'images (11), former un endoscope (10),
**caractérisée par** une partie de gaine proximale (70) munie d'une douille (71), la douille (71) se trouvant au niveau de la partie de gaine proximale (70) et la douille (71) étant reliée à la partie de gaine proximale (70) de manière rotative par le biais d'une fixation axiale (72),
l'arrangement à champ visuel (61) et la partie de gaine proximale (70) munie de la douille (71) étant configurés pour réaliser une liaison mécanique amovible entre l'arrangement à champ visuel (61) et la partie de gaine proximale (70) par le biais de la douille (71) et la fixation axiale (72) et pour former la gaine (60).

2. Gaine (60) selon la revendication précédente, avec laquelle le dispositif optique (87, 92) est configuré au moins soit pour réfracter, soit pour réfléchir, soit pour diffracter la lumière.

3. Gaine (60) selon l'une des revendications précédentes, avec laquelle le dispositif optique comporte au moins soit un prisme (87), soit un miroir, soit une lentille (92).

4. Gaine (60) selon l'une des revendications précédentes, comprenant en outre un dispositif d'ajustement (68, 71, 78) destiné à fixer de manière ajustable la position d'un arrangement de transmission d'images (11) dans le sens longitudinal dans le canal (90) .

5. Gaine (60) selon l'une des revendications précédentes, comprenant en outre une fibre optique (91) destinée à la transmission de lumière d'éclairage de l'extrémité proximale à l'extrémité distale de l'arrangement à champ visuel (61) en vue d'éclairer un objet à observer.

6. Gaine (60) selon l'une des revendications précédentes, comprenant en outre un dispositif de sortie de lumière (64) au niveau de l'extrémité distale de l'arrangement à champ visuel (61), servant à guider la lumière d'éclairage dans une zone de l'espace en-dehors de l'arrangement à champ visuel (61).

7. Gaine (60) selon l'une des revendications précédentes, avec laquelle le dispositif optique (87, 92) est rendu étanche aux fluides par rapport à l'extérieur ou par rapport à l'extérieur et par rapport au canal (90).

8. Endoscope (10) comprenant une gaine (60) selon l'une des revendications 1 à 7 et un arrangement de transmission d'images (11), l'arrangement de transmission d'images (11) pouvant être inséré dans le canal (90) de l'arrangement à champ visuel (61), l'arrangement de transmission d'images (11) possédant au moins soit un dispositif destiné à la transmission optique d'une image d'une extrémité distale à une extrémité proximale de l'endoscope (10), soit un capteur photosensible (41) destiné à convertir une image en un signal d'image.

9. Endoscope (10) selon la revendication précédente, l'arrangement de transmission d'images (11) comprenant :
une tige (20) qui est configurée pour être introduite dans le canal (90) de l'arrangement à champ visuel (61) ;
au moins soit un dispositif destiné à la transmission optique d'une image d'une extrémité distale (12) à une extrémité proximale (13) de la tige (20), soit un capteur photosensible (41) destiné à convertir une image en un signal d'image.
